# EUROPEAN PATENT APPLICATION

(11) **EP 4 406 970 A1**
(43) Date of publication of application: **31.07.2024**
(21) Application number: 22871431.7
(22) Date of filing: 23.05.2022
(51) Int. Cl.: C07K 16/28, C07K 16/46, C12N 5/16, C12N 15/13, C12N 15/63, A61K 39/395, A61P 31/00, A61P 35/00, A61P 37/04, G01N 33/577

(54) **MONOCLONAL ANTIBODY TARGETING TIGIT**

(30) Priority: 22.09.2021 CN 202111105544
(71) Applicant: Shanghai Celgen Bio-Pharmaceutical Co., Ltd., Shanghai 201203 (CN)
(72) Inventor: CHEN, Yi, Shanghai 201203 (CN); CAI, Zeling, Shanghai 201203 (CN); HE, Qiaoqiao, Shanghai 201203 (CN); WANG, Yaling, Shanghai 201203 (CN)
(74) Representative: Santoro, Sofia
(86) International application number: PCT/CN2022/094422
(87) International publication number: WO 2023/045370

(57) **Abstract**

Provided is an antibody or an antigen-binding fragment thereof that binds to human TIGIT (T cell immunoreceptor with immunoglobulin and ITIM domains). Also provided are a nucleic acid molecule encoding the antibody or the antigen-binding fragment thereof, a vector and host cell containing the nucleic acid molecule, an immunoconjugate and composition comprising the antibody or the antigen-binding fragment thereof, and the use of the antibody or the antigen-binding fragment thereof in the preparation of a product for positively adjusting the activity of immune cells and/or improving the immune response, a product for reducing or eliminating the immunosuppressive effect of cells expressing TIGIT (such as Treg cells), and a kit for detecting the presence or absence of TIGIT and the level or activity of TIGIT.

## Description

### Technical field

The present disclosure belongs to the fields of biotechnology and medicine. Specifically, the present disclosure relates to an antibody or an antigen-binding fragment thereof that binds to human TIGIT (T cell immunoreceptor with immunoglobulin and ITIM domains), and a nucleic acid molecule thereof, a vector and host cell containing the nucleic acid molecule, an immunoconjugate and composition comprising the antibody or the antigen-binding fragment thereof, and use of the aforementioned products.

### Background

T cells are key mediators of immune response, and their activation depends on TCR signals and costimulatory signals. In an immune response, some of the costimulatory signals exert a forward stimulation function, and some perform a negative regulation function, that is, the immune response is positively adjusted (for example, OX40, GITR, and 4-1BB) of the costimulatory molecule, or is subjected to negative regulation of the immune response checkpoint molecules (such as PD-1 and CTLA-4) (Rudd C. E., Unifying concepts in CD28, ICOS, and CTLA4 co-receptor signaling. Nat Rev Immunol 3:544-556, 2003; Rudd C E. The reverse stop-signal model for CTLA4 function, Nat Rev Immunol 8:153-160, 2008). Inhibitory costimulation can prevent the onset of immune responses or down-regulate immune responses. However, it also limits the ability of the immune system to combat cancer and infectious diseases.

Tumor immunotherapy is a leading edge and hot field of tumor treatment foundation and clinic research in recent years. The first generation immune checkpoint inhibitor represented by CTLA-4 and PD-1/PD-L1 monoclonal antibody can exert an anti-tumor effect by activating a T cell-mediated immune response. However, the first-generation immune checkpoint antibody still faces the problems of limited patients, poor tumor effect, drug tolerance and the like. At the same time, a significant portion of the early-treated effective patient may have a tumor recurrence condition during or after treatment. In order to solve the above problems, it is crucial to find and verify new immune checkpoint inhibitory molecules.

A variety of new immune checkpoint molecules have been reported, such as TIM-3, LAG-3, TIGIT, BTLA, and VISTA (Mercier et al., Front. Immunol. 6: 418, 2015). T cell immunoreceptor with Ig and ITIM domains (TIGIT, also known as WURAM, VSTM3 or VSIG 9), is a checkpoint molecule mainly expressed on the surface of immune cells such as NK cells, T cells, Treg cells, etc., which has an immunoreceptor tyrosine-based inhibitory motif (ITIM) at the cytoplasmic tail, and is a typical inhibitory receptor protein (Yu et al., Nat. Immunol. 10: 48-57, 2009).

It is known that TIGIT may interact with CD155 (also referred to as PVR and necl-5), CD 112 (also referred to as PVRL2 and laminin-2) and possibly with CD113 (also referred to as PVRL 3 and laminin-3), wherein CD155 is a high affinity core ligand of TIGIT, which interacts with TIGIT much higher than other paired molecules in the pathway (Martinet et al. Nat. Rev. Immunol. 15: 243-254, 2015).

TIGIT may inhibit the immune response of the body through a variety of mechanisms. By targeting a signal pathway molecule of a T cell antigen receptor (TCR), TIGIT directly blocks the acquisition of initial T cell (Tn) activation, proliferation and effector functions, and can inhibit cell proliferation and inflammatory cytokine production of CD4+ T cells (Forurcade J. et al., JCI Insight, 2018, 3 (14):e121157). TIGIT can indirectly inhibit T cells by adjusting cytokine production of dendritic cells (Yu et al., Nat. Immunol. 10: 48-57, 2009). TIGIT can also enhance the Tregs stability and its inhibitory function on the proliferation of T cells that produce IFN-γ (Fourcade J. et al., JCI Insight, 2018, 3 (14): e121157). In a tumor environment, TIGIT is highly expressed in tumor infiltration or migration-related NK cells and effector T cell surfaces, CD155 is highly expressed on the surface of tumor cells, tumor cells directly act on NK cells and effector T cells via binding of CD155/TIGIT, thereby inhibiting their activity (Li et al., J Biol. Chem. 289: 17647-17657, 2014).

In summary, TIGIT is an extremely potential immunotherapy new target for enhancing immune response and preventing and/or treating tumors, infections or infectious diseases. CD155 blocking antibodies targeting TIGIT, especially anti-human TIGIT antibodies, can release tumor killing activity of immune effector cells, and are expected to achieve good anti-tumor efficacy, and indications include small cell lung cancer, esophageal cancer, esophageal squamous cell cancer, non-small cell lung cancer, head and neck squamous cell cancer, liver cancer, cervical cancer, triple negative breast tumor, B-cell lymphoma, multiple bone marrow cancer, non-Hodgkin lymphoma, melanoma, ovarian cancer, colon cancer, breast cancer, and the like.

At present, there are several TIGIT antibodies in clinical trials, including Roche's Tiragolumab, which has entered phase III clinical trials. However, at present, there is no TIGIT-blocking monoclonal antibody drugs ton the market at home and abroad. Therefore, it is necessary to develop TIGIT monoclonal antibodies with high affinity, high specificity and better effect.

### SUMMARY OF THE INVENTION

The present disclosure provides a TIGIT monoclonal antibody with high affinity, specificity, and superior efficacy.

In some aspects of the present disclosure, it provides a monoclonal antibody or antigen-binding fragment thereof targeting T cell immunoglobulin and ITIM domain protein (TIGIT), which competes with CD155 to bind to TIGIT.

In some aspects of the present disclosure, it provides a monoclonal antibody or antigen-binding fragment thereof targeting T cell immunoglobulin and ITIM domain protein (TIGIT), comprising specific heavy chain complementary determining regions (VH CDR) 1-3 and light chain complementary determining regions (VL CDR) 1-3 disclosed in the present disclosure, or a sequence having at least 95% sequence identity thereto.

In some aspects of the present disclosure, it provides a nucleic acid molecule encoding the monoclonal antibody or the antigen-binding fragment thereof of the present disclosure, or a vector or a host cell containing the nucleic acid molecule.

In some aspects of the present disclosure, it provides an immunoconjugate comprising the monoclonal antibody or the antigen-binding fragment thereof.

In some aspects of the present disclosure, it provides a composition comprising the monoclonal antibody or antigen-binding fragment thereof, a nucleic acid molecule, a vector, or a host cell or an immunoconjugate of the present disclosure.

In some aspects of the present disclosure, it provides a chimeric antigen receptor, comprising an extracellular domain and an intracellular domain, wherein the extracellular domain comprises the monoclonal antibody or antigen-binding fragment thereof of the present application.

In some aspects of the present disclosure, it provides a nucleic acid molecule and a construct or vector comprising the nucleotide molecule, wherein the nucleic acid molecule comprises a coding sequence of the chimeric antigen receptor of the present application.

In some aspects of the present disclosure, it provides a transformed immune cell, which expresses the chimeric antigen receptor of the present application, or is transformed using a nucleic acid molecule, construct, or vector of the present application, for example, the immune cell is selected from T cells, such as αβ T cells, γδ T cells or NK T cells or T cells derived from pluripotent cells.

In some aspects of the present disclosure, it provides a use of the monoclonal antibody or antigen binding fragment thereof of the present disclosure, the nucleic acid molecule, the vector or host cell, the immunoconjugate, the chimeric antigen receptor of the present disclosure or the encoding nucleic acid molecule thereof, the construct or vector, the transformed immune cell and/or the composition of the present disclosure in preparation of a product for positively regulating immune cell activity and/or improving immune response, such as use of the preparation of a medicine for preventing and/or treating tumor, infection or infectious disease.

In some aspects of the present disclosure, it provides a method for positively regulating immune cell activity and/or enhancing immune response, which comprises administering the monoclonal antibody or antigen-binding fragment thereof in the present disclosure, nucleic acid molecule, vector or host cell, immunoconjugate, chimeric antigen receptor or encoded nucleic acid molecule thereof, construct or vector, transformed immune cells, and/or composition of the present disclosure to a subject in need, for example, for preventing and/or treating tumor, infection or infectious disease.

In some aspects of the present disclosure, it provides a use of the composition of the monoclonal antibody or antigen binding fragment thereof of the present disclosure, the nucleic acid molecule, the vector or host cell, the immunoconjugate, the chimeric antigen receptor of the present disclosure or the encoding nucleic acid molecule thereof, the construct or vector, the transformed immune cells and/or the composition of the present disclosure in positively regulating immune cell activity and/or improving immune response (such as for preventing and/or treating tumor, infection or infectious diseases).

In some aspects of the present disclosure, it provides a use of he monoclonal antibody or antigen binding fragment thereof of the present disclosure, the nucleic acid molecule, the vector or host cell, the immunoconjugate, the chimeric antigen receptor of the present disclosure or the encoding nucleic acid molecule thereof, the construct or vector, the transformed immune cells and/or the composition of the present disclosure in preparation of a product for reducing or eliminating the ells expressing TIGIT (such as Treg cells).

In some aspects of the present disclosure, it provides a method for reducing or eliminating the cells expressing TIGIT (such as Treg cells), which includes administering the monoclonal antibody or antigen binding fragment thereof of the present disclosure, the nucleic acid molecule, the vector or host cell, the immunoconjugate, the chimeric antigen receptor of the present disclosure or the encoding nucleic acid molecule thereof, the construct or vector, the transformed immune cells and/or the composition of the present disclosure to a subject in need.

In some aspects of the present disclosure, it provides a use of the monoclonal antibody or antigen binding fragment thereof of the present disclosure, the nucleic acid molecule, the vector or host cell, the immunoconjugate, the chimeric antigen receptor of the present disclosure or the encoding nucleic acid molecule thereof, the construct or vector, the transformed immune cells and/or the composition of the present disclosure in reducing or eliminating the ells expressing TIGIT (such as Treg cells).

Those skilled in the art can arbitrarily combine the foregoing technical solutions and technical features without departing from the spirit and scope of the present disclosure. Other aspects of the disclosure will be apparent to those skilled in the art from the disclosure herein.

### DESCRIPTION OF THE DRAWINGS

The present disclosure is further described below with reference to the accompanying drawings, wherein these displays are merely illustrative of embodiments of the present disclosure, and are not intended to limit the scope of the present disclosure.
Fig. 1 shows the SDS-PAGE electrophoresis analysis of 8 strains of anti-human TIGIT mouse monoclonal antibodies (NO.:7103-01, 7103-02, 7103-03, 7103-04, 7103-05, 7105-06, 7103-07, 7103-08): top: non-reduced 4-12% SDS-PAGE electrophoretic analysis; bottom: reduced 4-12% SDS-PAGE electrophoretic analysis. The molecular weight of the non-reduced antibody is about 150 kD, and the molecular weight of the reduced antibody is about 50 kD and about 25 kD, respectively.
Fig. 2 shows the ELISA study of 8 strains of anti-human TIGIT mouse monoclonal antibodies (numbered 7103-01, 7103-02, 7103-03, 7103-04, 7103-05, 7105-06, 7103-07, 7103-08) for binding to recombinant human TIGIT *in vitro.*
Fig. 3 shows the competitive ELISA study of 8 anti-human TIGIT mouse monoclonal antibodies (numbered 7103-01, 7103-02, 7103-03, 7103-04, 7103-05, 7105-06, 7103-07, 7103-08) and CD155 for binding to recombinant human TIGIT.
Fig. 4 shows the ELISA study of 8 strains of anti-human TIGIT mouse monoclonal antibodies (numbered 7103-01, 7103-02, 7103-03, 7103-04, 7103-05, 7105-06, 7103-07, 7103-08) for binding to recombinant cynomolgus TIGIT *in vitro.*
Fig. 5 shows the flow cytometric analysis study of the binding of 5 strains of anti-human TIGIT mouse monoclonal antibodies (numbered 7103-01, 7103-03, 7103-04, 7105-06, 7103-07) to membrane TIGIT from CHO cells overexpressing TIGIT.
Fig. 6 shows the SDS-PAGE electrophoresis analysis of 5 strains of anti-human TIGIT human mouse chimeric monoclonal antibodies (numbered 7103-01 (hFc), 7103-03 (hFc), 7103-04 (hFc), 7103-06 (hFc), 7103-07 (hFc)), wherein the molecular weight of the non-reduced antibody is about 150 kDa, and the molecular weight of the reduced antibody is about 55 kDa and about 25 kDa, respectively.
Fig. 7 shows the ELISA study of 5 strains of anti-human TIGIT human mouse chimeric monoclonal antibodies (numbered 7103-01 (hFc), 7103-03 (hFc), 7103-04 (hFc), 7103-05 (hFc), 7103-06 (hFc), 7103-07 (hFc)) for binding to recombinant human TIGIT *in vitro.*
Fig. 8 shows the flow cytometry analysis of 5 human mouse chimeric TIGIT monoclonal antibodies (numbered 7103-01 (hFc), 7103-03 (hFc), 7103-04 (hFc), 7103-05 (hFc), 7103-06 (hFc), 7103-07 (hFc)) for binding to membrane TIGIT.
Fig. 9 shows the competitive ELISA study of 5 human mouse chimeric TIGIT monoclonal antibodies (numbered 7103-01 (hFc), 7103-03 (hFc), 7103-04 (hFc), 7103-05 (hFc), 7103-06 (hFc), 7103-07 (hFc)) and CD155 for binding to recombinant human TIGIT.

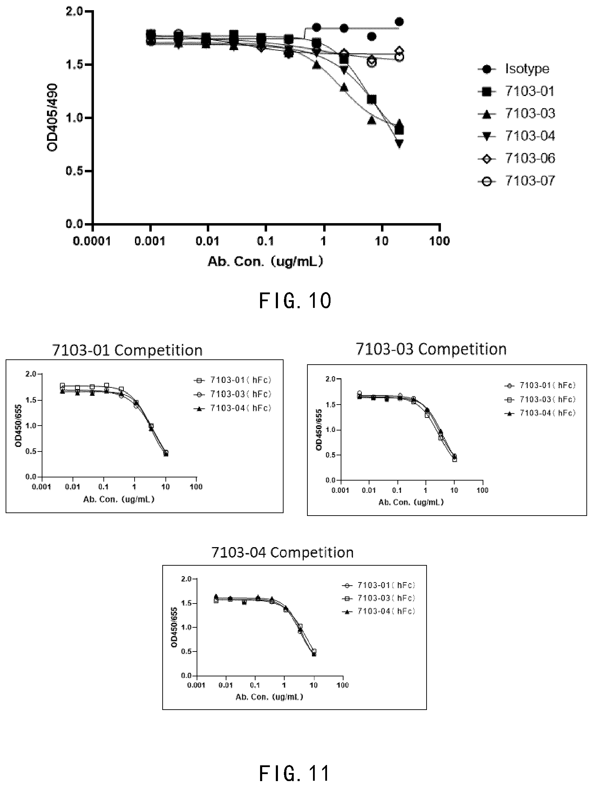

Fig. 10 shows the competitive ELISA study of anti-human TIGIT mouse monoclonal antibodies (numbered 7103-01, 7103-03, 7103-04, 7105-06, 7103-07) for binding recombinant human TIGIT in competition with Tiragolumab. The isotype in the figure is the negative control of isotype antibody.
Fig. 11 shows the competitive ELISA study of 3 anti-human TIGIT mouse monoclonal antibodies (numbered 7103-01, 7103-03, 7103-04) for binding to recombinant human TIGIT in competition with human mouse chimeric TIGIT monoclonal antibodies 7103-01 (hFc), 7103-03 (hFc), and 7103-04 (hFc), respectively.
Fig. 12 shows the promotion effect of 5 human mouse chimeric TIGIT monoclonal antibodies (numbered 7103-01 (hFc), 7103-03 (hFc), 7103-04 (hFc), 7103-05 (hFc), 7103-06 (hFc), 7103-07 (hFc)) on PHA-activated PBMC to produce IFNγ.
Fig. 13 shows the ELISA study of 6 strains of humanized 7103-01 anti-human TIGIT monoclonal antibodies (numbered P03486, P03487, P03490, P03491, P03492, P03493) for binding to recombinant human TIGIT *in vitro.* Human mouse chimeric 7301-01 (hFc) antibodies and Tiragolumab are used as positive controls.
Fig. 14A shows the ELISA study of 9 strains of 7103-07 anti-human TIGIT humanized monoclonal antibodies (numbered P03476, P03477, P03478, P03479, P03480, P03481, P03482, P03483, and P03484) for binding to recombinant human TIGIT *in vitro.* Human mouse chimeric 7303-07 (hFc) antibodies and Tiragolumab are used as positive controls.
Fig. 14B shows the ELISA study of another batch of 5 strains of 7103-07 anti-human TIGIT humanized monoclonal antibodies (numbered P04662, P04663, P04664, P04665, P04666, and P04667) for binding to recombinant human TIGIT *in vitro.* Human mouse chimeric antibodies 7103-07 (hFc) and Tiragolumab (also known as Tira) are used as positive controls.
Fig. 15 shows the flow cytometry analysis of 3 strains of 7103-01 anti-human TIGIT humanized monoclonal antibodies (numbered P03489, P03492, P03493) for binding to membrane TIGIT.
Fig. 16 shows the flow cytometry analysis of 9 strains of 7103-07 anti-human TIGIT humanized monoclonal antibodies (numbered P03476, P03477, P03478, P03479, P03480, P03481, P03482, P03483) for binding to membrane TIGIT.
Fig. 17A shows the competitive ELISA study of 9 strains of 7103-07 anti-human TIGIT humanized monoclonal antibodies (numbered P03476, P03477, P03478, P03479, P03480, P03481, P03482, P03483, and P03484) and CD155 for binding to recombinant human TIGIT.
Fig. 17B shows the competitive ELISA study of another batch of 4 strains of 7103-07 anti-human TIGIT humanized monoclonal antibody numbers (numbered P04662, P04663, P04666, and P04667) and CD155 in binding to recombinant human TIGIT.
Fig. 18 shows the promotion effect of 7 humanized TIGIT monoclonal antibodies (numbered P03479, P03480, P03481, P03488, P03489, P03491, and P03493) on PHA-activated PBMC to produce IFNγ.
Fig. 19 shows the effect of anti-human TIGIT humanized monoclonal antibody in inhibiting tumor growth in mice:
   Fig. 19A: relationship between tumor volume and time for each mouse in each group;
   Fig. 19B: tumor weight of each mouse in each group at the end of the experiment (D17).

In the above figures, * represents p<0.05 and * * represents p<0.01.

### Detailed Description

The present disclosure provides antibodies, antibody fragments, and related compositions and molecules that can specifically bind to human TIGIT. Furthermore, the present disclosure relates to murine antibodies, human-mouse chimeric antibodies, and humanized antibodies of the TIGIT antibody. This disclosure also relates to pharmaceutical compositions containing the TIGIT antibody, as well as their use as diagnostic and therapeutic drugs for related diseases. The anti-TIGIT antibody of the present disclosure has high specificity and high affinity to human TIGIT, can effectively block the combination of TIGIT and its ligand CD155, enhance the release of cytokines of immune cells, improve the activity of immune cells, enhance immune response, and is useful to prevent and/or treat tumors, infections or infectious diseases. The anti-TIGIT antibodies of the present disclosure can also reduce or eliminate cells expressing TIGIT (such as Treg cells) or their activity.

The present disclosure also provides a method for enhancing immune response, preventing and/or treating tumors, infections or infectious diseases, which comprises administering to a subject a therapeutically effective amount of a pharmaceutical composition described herein, wherein the pharmaceutical composition blocks the binding of human TIGIT to ligand CD155.

All numerical ranges provided herein are intended to clearly include all numerical values falling between range endpoints and numerical ranges therebetween. The features mentioned in the present disclosure or the features mentioned in the examples can be combined. All features disclosed in this specification can be combined with any combination form, and the various features disclosed in the specification can be substituted by any alternative feature that can provide the same, equal, or similar purpose. Therefore, unless otherwise specified, the disclosed features are only general examples of equal or similar features.

As used herein, "comprising", "having" or "including" includes "containing", "consisting mainly of", "consisting essentially of", and "consisting of". The terms "consisting mainly of", "consisting essentially of" and "consisting of" are subordinate concepts of "comprising", "having" or "including".

### Monoclonal antibodies and preparation thereof

As used herein, the term "monoclonal antibody (mAb)" refers to an antibody obtained from a substantially homogeneous population, i.e., the individual antibodies contained in the population are the same, except for a few naturally occurring mutations that may be present. A monoclonal antibody is highly specific for a single antigenic site. Moreover, unlike conventional polyclonal antibody preparations (usually with different antibodies directed against different determinants), each monoclonal antibody is directed against a single determinant on the antigen. In addition to their specificity, the benefit of monoclonal antibodies is that they are synthesized by hybridoma culture and are not contaminated with other immunoglobulins. The modifier "monoclonal" indicates the properties of the antibody and is obtained from a homogeneous antibody population, which should not be interpreted as requiring any special method to produce the antibody.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the term "variable" means that certain portion of the variable region in an antibody differ in sequence, which is responsible for the binding and specificity of various specific antibodies to their specific antigen. However, the variability is not distributed evenly throughout the variable regions of an antibody. It is concentrated in three fragments called complementarity determination regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partical β-sheet structure. The CDRs in each chain are closely linked together via the FR regions, and together with the CDRs of the other chain, form the antigen binding site of an antibody (see Kabat et al., NIH Publ. No. 91-3242, Volume I, pages 647-669 (1991)). Constant regions are not directly involved in the binding of antibodies to antigen. However, they exhibit different effector functions, such as participating in the antibody-dependent cytotoxicity of antibodies.

This disclosure provides antibodies that specifically bind to human TIGIT, including murine antibodies, chimeric antibodies, and humanized antibodies. The monoclonal antibodies of the present disclosure can also be chimeric antibodies or humanized antibodies. In the present disclosure, unless otherwise specified, "m" represents the mouse derived portion and "h" represents the human derived portion. In some embodiments, the antibody comprises a heavy chain variable region (VH), which comprises 3 complementary determining regions (CDRs), and the antibody comprises a light chain variable region (VL)which comprises 3 CDRs.

As used herein, the term "sequence identity" or "identity%" refers to the percentage of the same residues (such as amino acids or nucleic acids) between the candidate sequence and the reference sequence after aligning the sequence and (if necessary) introducing vacancies to obtain the maximum sequence identity percentage. For example, as used herein, " at least 70% sequence identity " means that the sequence identity between the candidate sequence and the reference sequence reaches 70% or more, such as 75, 80, 85, 90, 91, 92, 93, 94, 95, 96, 97, 98, 99, 100%, or any numerical point identity therein.

In some embodiments, the VH amino acid sequence of the antibody is selected from SEQ ID NO: 27, 35, 43, 51, 59, 67, 75, 83, 124, 125, 126, 127, 131, 132, 134, 135, 136, or a sequence having at least 70% sequence identity thereto.

In some embodiments, the VL amino acid sequence of the antibody is selected from: SEQ ID NO: 31, 39, 47, 55, 63, 71, 79, 87, 129, 130, 137, 138, 139, 140, 141, or a sequence having at least 70% sequence identity thereto.

In some embodiments, the antibody or antigen binding fragment thereof comprises VH CDR1 selected from the following group consisting of: 28, 36, 44, 52, 60, 68, 76, and 84; VH CDR2 selected from the following group consisting of: 29, 37, 45, 53, 61, 69, 77, 85, 128, 133, 142, and 143. In some embodiments, the antibody or antigen binding fragment thereof comprises VH CDR3 selected from the following group consisting of: 30, 38, 46, 54, 62, 70, 78, and 86. In some embodiments, the antibody or antigen binding fragment thereof comprises VL CDR1 selected from the following group consisting of: 32, 40, 48, 56, 64, 72, 80, and 88. In some embodiments, the antibody or antigen binding fragment thereof comprises VL CDR2 selected from the following group consisting of: 33, 41, 49, 57, 65, 73, 81, and 89. In some embodiments, the antibody or antigen binding fragment thereof comprises VL CDR3 selected from the following group consisting of: 34, 42, 50, 58, 66, 74, 72, and 90.

In some embodiments, the CDR sequence of the antibody may be modified to meet requirements such as humanization. For example, the modified CDR may have at least 90%-99% or higher sequence identity to the parent sequence. For example, modified CDR may have 1, 2, or 3 amino acid residues from the parent sequence. For example, one or more of VH CDR1, VH CDR2, VH CDR3, VL CDR1, VL CDR2, and VL CDR3 contain altered amino acid residues. In some embodiments, VH CDR2 is modified. For example, in some embodiments, the VH CDR2 of SEQ ID NO: 29 is replaced by a sequence with SEQ ID NO: 128 or SEQ ID NO: 142; For example, in some embodiments, the VH CDR2 of SEQ ID NO: 77 is replaced by a sequence with SEQ ID NO: 133 or SEQ ID NO: 143.

In some embodiments, different combinations of CDR sequences disclosed herein may be used to obtain antibody or antigen binding fragments with desired binding and/or targeting and/or activity. In some embodiments, VL CDR2 of SPSYRYT (SEQ ID NO: 33, 57, and 65) can be combined with VL CDR1 and VL CDR3 of SEQ ID NO: 32 and 34, or SEQ ID NO: 56 and 58, or SEQ ID NO: 64 and 66. In some embodiments, VL CDR3 of QQHYSTPWT (SEQ ID NO: 34, 50, and 66) can be combined with VL CDR1 and VL CDR2 of SEQ ID NO: 32 and 33, or SEQ ID NO: 48 and 49, or SEQ ID NO: 64 and 65. In some embodiments, VH CDR1 of TYAMS (SEQ ID NO: 68 and 76), VH CDR3 of KTLDYYALDY (SEQ ID NO: 70 and 78), VL CDR2 of SGSRLQS (SEQ ID NO: 73 and 81), and VL CDR3 of QQHNEYPWT (SEQ ID NO: 74 and 82) can be combined with VH CDR2 of SEQ ID NO: 69 or 77 and VH CDR1 of SEQ ID NO: 72 or 80.

In some embodiments, the antibody comprises any combination of VH amino acid sequences and VL amino acid sequences selected from the above. In some embodiments, the amino acid sequence combinations of VH and VL of the antibody are: SEQ ID NO: 27 and SEQ ID NO: 31; SEQ ID NO: 35 and 39; SEQ ID NO: 43 and 47; SEQ ID NO: 51 and 55; SEQ ID NO: 59 and 63; SEQ ID NO: 67 and 71; SEQ ID NO: 75 and 79; SEQ ID NO: 83 and 87; the combination of any humanized VH selected from SEQ ID NO: 124-126 and any VL sequence selected from SEQ ID NO: 31, 39, 47, 55, 63, 71, 79, 87, 129, 130, 137, 138, 139, 140, and 141; the combination of any VH sequence selected from SEQ ID NO: 27, 35, 43, 51, 59, 67, 75, 83, 124, 125, 126, 127, 131, 132, 134, 135, and 136 and any humanized VL sequence selected from SEQ ID NO: 129, 130, 137, 138, 139, 140, and 141.

In some embodiments, the amino acid sequences of VH CDR1, 2, and 3 of the antibody are SYNIY (SEQ ID NO: 28), GVNPSNGNTNFNENFKS (SEQ ID NO: 29), and GNYYGYEFAY (SEQ ID NO: 30), respectively. In some embodiments, the amino acid sequences of VL CDR1, 2, and 3 of the antibody are KASQHVSTAVA (SEQ ID NO: 32), SPSYRYT (SEQ ID NO: 33), and QQHYSTPWT (SEQ ID NO: 34), respectively.

In some embodiments, the VH amino acid sequence of the antibody is SEQ ID NO: 27 or a sequence having at least 70% sequence identity thereto, and the amino acid sequences of VH CDR1, 2, and 3 of the antibody are SYNIY (SEQ ID NO: 28), GVNPSNGNTNFNENFKS (SEQ ID NO: 29), and GNYYGYEFAY (SEQ ID NO: 30), respectively. In some embodiments, the VL amino acid sequence of the antibody is SEQ ID NO: 31 or a sequence having at least 70% sequence identity thereto. The amino acid sequences of the VL CDR1, 2, and 3 of the antibody are KASQHVSTAVA (SEQ ID NO: 32), SPSYRYT (SEQ ID NO: 33), and QQHYSTPWT (SEQ ID NO: 34), respectively.

In some embodiments, the amino acid sequences of VH CDR1, 2, and 3 of the antibody are SYVMH (SEQ ID NO: 36), YINPYNDGTKYNEKFKG (SEQ ID NO: 37), and WNPYYYAMDY (SEQ ID NO: 38), respectively. In some embodiments, the amino acid sequences of VL CDR1, 2, and 3 of the antibody are SANSSARYVH (SEQ ID NO: 40), EISKLAS (SEQ ID NO: 41), and QQWNYPLIT (SEQ ID NO: 42), respectively.

In some embodiments, the VH amino acid sequence of the antibody is SEQ ID NO: 35 or a sequence having at least 70% sequence identity thereto, while the amino acid sequences of CDR1, 2, and 3 are SYVMH (SEQ ID NO: 36), YINPYNDGTKYNEKFKG (SEQ ID NO: 37), and WNPYYYAMDY (SEQ ID NO: 38), respectively. The amino acid sequence of VL is SEQ ID NO: 39 or a sequence having at least 70% sequence identity thereto. The amino acid sequences of CDR1, 2, and 3 are SANSSARYVH (SEQ ID NO: 40), EISKLAS (SEQ ID NO: 41), and QQWNYPLIT (SEQ ID NO: 42), respectively.

In some embodiments, the amino acid sequences of VH CDR1, 2, and 3 of the antibody are EYTMH (SEQ ID NO: 44), GINPNNGGTSYNQKFKG (SEQ ID NO: 45), and GAYYRYDFDY (SEQ ID NO: 46), respectively. In some embodiments, the amino acid sequences of the VL CDR1, 2, and 3 of the antibody are KASQDVRTAVV (SEQ ID NO: 48), SASYRYT (SEQ ID NO: 49), and QQHYSTPWT (SEQ ID NO: 50), respectively.

In some embodiments, the VH amino acid sequence of the antibody is SEQ ID NO: 43 or a sequence having at least 70% sequence identity thereto, while the amino acid sequences of CDR1, 2, and 3 are EYTMH (SEQ ID NO: 44), GINPNNGGTSYNQKFKG (SEQ ID NO: 45), and GAYYRYDFDY (SEQ ID NO: 46), respectively. The amino acid sequence of VL is SEQ ID NO: 47 or a sequence having at least 70% sequence identity thereto, while the amino acid sequences of CDR1, 2, and 3 are KASQDVRTAVV (SEQ ID NO: 48), SASYRYT (SEQ ID NO: 49), and QQHYSTPWT (SEQ ID NO: 50), respectively.

In some embodiments, the amino acid sequences of VH CDR1, 2, and 3 of the antibody are DYFVY (SEQ ID NO: 52), GINPSNGGATFNEKFKN (SEQ ID NO: 53), and GNFYGYEFAY (SEQ ID NO: 54), respectively. In some embodiments, the amino acid sequences of VL CDR1, 2, and 3 of the antibody are KASQHVSTAVV (SEQ ID NO: 56), SPSYRYT (SEQ ID NO: 57), and QQHYITPWT (SEQ ID NO: 58), respectively.

In some embodiments, the VH amino acid sequence of the antibody is SEQ ID NO: 51 or a sequence having at least 70% sequence identity thereto, and the amino acid sequences of CDR1, 2, and 3 are DYFVY (SEQ ID NO: 52), GINPSNGGATFNEKFKN (SEQ ID NO: 53), and GNFYGYEFAY (SEQ ID NO: 54), respectively. The amino acid sequence of VL is SEQ ID NO: 55 or a sequence having at least 70% sequence identity thereto, and the amino acid sequences of CDR1, 2, and 3 are KASQHVSTAVV (SEQ ID NO: 56), SPSYRYT (SEQ ID NO: 57), and QQHYITPWT (SEQ ID NO: 58), respectively.

In some embodiments, the amino acid sequences of VH CDR1, 2, and 3 of the antibody are EYTMY (SEQ ID NO: 60), GINPNNGGTRYNQKFKG (SEQ ID NO: 61), and GGHYDYGFAY (SEQ ID NO: 62), respectively. In some embodiments, the amino acid sequences of VL CDR1, 2, and 3 of the antibody are KASQDVKTAVA (SEQ ID NO: 64), SPSYRYT (SEQ ID NO: 65), and QQHYSTPWT (SEQ ID NO: 66), respectively.

In some embodiments, the VH amino acid sequence of the antibody is SEQ ID NO: 59 or a sequence having at least 70% sequence identity thereto, and the amino acid sequences of CDR1, 2, and 3 are EYTMY (SEQ ID NO: 60), GINPNNGGTRYNQKFKG (SEQ ID NO: 61), and GGHYDYGFAY (SEQ ID NO: 62), respectively. The amino acid sequence of VL is SEQ ID NO: 63 or a sequence having at least 70% sequence identity thereto, and the amino acid sequences of CDR1, 2, and 3 are KASQDVKTAVA (SEQ ID NO: 64), SPSYRYT (SEQ ID NO: 65), and QQHYSTPWT (SEQ ID NO: 66), respectively.

In some embodiments, the amino acid sequences of VH CDR1, 2, and 3 of the antibody are TYAMS (SEQ ID NO: 68), EISSGGSYTYYSDTVTG (SEQ ID NO: 69), and KTLDYYALDY (SEQ ID NO: 70), respectively. In some embodiments, the amino acid sequences of VL CDR1, 2, and 3 of the antibody are RANKTISKYLA (SEQ ID NO: 72), SGSRLQS (SEQ ID NO: 73), and QQHNEYPWT (SEQ ID NO: 74), respectively.

In some embodiments, the VH amino acid sequence of the antibody is SEQ ID NO: 67 or a sequence having at least 70% sequence identity thereto, and the amino acid sequences of CDR1, 2, and 3 are TYAMS (SEQ ID NO: 68), EISSGGSYTYYSDTVTG (SEQ ID NO: 69), and KTLDYYALDY (SEQ ID NO: 70), respectively. The amino acid sequence of VL is SEQ ID NO: 71 or a sequence having at least 70% sequence identity thereto, and the amino acid sequences of CDR1, 2, and 3 are RANKTISKYLA (SEQ ID NO: 72), SGSRLQS (SEQ ID NO: 73), and QQHNEYPWT (SEQ ID NO: 74), respectively.

In some embodiments, the amino acid sequences of VH CDR1, 2, and 3 of the antibody are TYAMS (SEQ ID NO: 76), EISSGGSHTFYSDTVTG (SEQ ID NO: 77), and KTLDYYALDY (SEQ ID NO: 78), respectively. In some embodiments, the amino acid sequences of VL CDR1, 2, and 3 of the antibody are RASKSISKYLA (SEQ ID NO: 80), SGSRLQS (SEQ ID NO: 81), and QQHNEYPWT (SEQ ID NO: 82), respectively.

In some embodiments, the VH amino acid sequence of the antibody is SEQ ID NO: 75 or a sequence having at least 70% sequence identity thereto, and the amino acid sequences of CDR1, 2, and 3 are TYAMS (SEQ ID NO: 76), EISSGGSHTFYSDTVTG (SEQ ID NO: 77), and KTLDYYALDY (SEQ ID NO: 78), respectively. The amino acid sequence of VL is SEQ ID NO: 79 or a sequence having at least 70% sequence identity thereto,and the amino acid sequences of CDR1, 2, and 3 are RASKSISKYLA (SEQ ID NO: 80), SGSRLQS (SEQ ID NO: 81), and QQHNEYPWT (SEQ ID NO: 82), respectively.

In some embodiments, the amino acid sequences of VH CDR1, 2, and 3 of the antibody are GYYML (SEQ ID NO: 84), RINPYNGATTYNQNFKD (SEQ ID NO: 85), and WFGDFDF (SEQ ID NO: 86), respectively. In some embodiments, the amino acid sequences of the VL CDR1, 2, and 3 of the antibody are RASQEISGYLS (SEQ ID NO: 88), ATSTLDS (SEQ ID NO: 89), and LQYASYPFT (SEQ ID NO: 90), respectively.

In some embodiments, the VH amino acid sequence of the antibody is SEQ ID NO: 83 or a sequence having at least 70% sequence identity thereto, and the amino acid sequences of CDR1, 2, and 3 are GYYML (SEQ ID NO: 84), RINPYNGATTYNQNFKD (SEQ ID NO: 85), and WFGDFDF (SEQ ID NO: 86), respectively. The amino acid sequence of VL is SEQ ID NO: 87 or a sequence having at least 70% sequence identity thereto, and the amino acid sequences of CDR1, 2, and 3 are RASQEISGYLS (SEQ ID NO: 88), ATSTLDS (SEQ ID NO: 89), and LQYASYPFT (SEQ ID NO: 90), respectively.

In some embodiments, the antibody is humanized and its VH amino acid sequence is any of the heavy chain variable regions shown in SEQ ID NO: 124, 125, 126, 127, and the amino acid sequences of CDR1, 2, and 3 are CDR1: SYNIY (SEQ ID NO: 28), CDR2: GVNPSNGNTNFNENFKS (SEQ ID NO: 29), or GVNPSNGNTNFNENFKG (SEQ ID NO: 142) or GVNPSNGNTNFNENFQG (SEQ ID NO: 128) and CDR3: GNYYGYEFAY (SEQ ID NO: 30).

In some embodiments, the antibody is humanized and its VL amino acid sequence is any of the light chain variable regions shown in SEQ ID NO: 129, 130, and the amino acid sequences of CDR1, 2, and 3 are KASQHVSTAVA (SEQ ID NO: 32), SPSYRYT (SEQ ID NO: 33), and QQHYSTPWT (SEQ ID NO: 34), respectively.

In some embodiments, the antibody is humanized and its VH amino acid sequence is any of the heavy chain variable regions shown in SEQ ID NO: 131, 132, 134, 135, or 136, and the amino acid sequences of CDR1, 2, and 3 are CDR1: TYAMS (SEQ ID NO: 76), CDR2: EISSGGSHTFYSDTVTG (SEQ ID NO: 77), or EISSGGSHTFYADTVKG (SEQ ID: 133) or EISSGGSHTFYADTVTG (SEQ ID NO: 143), and CDR3: KTLDYYALDY (SEQ ID NO: 78).

In some embodiments, the antibody is humanized and its VL amino acid sequence is any of the light chain variable regions shown in SEQ ID NO: 137, 138, 139, 140, or 141, and the amino acid sequences of CDR1, 2, and 3 are RASKSISKYLA (SEQ ID NO: 80), SGSRLQS (SEQ ID NO: 81), and QQHNEYPWT (SEQ ID NO: 82), respectively.

In some embodiments, a non-human monoclonal antibody may be humanized by (1) homologous substitution, where a human FR with greater homology to the non-human counterpart is used for substitution; (2) surface remodeling, where the surface amino acid residues of the non-human CDRs and FRs are remodeled to resemble the profile of the CDRs of the human antibody or the FR pattern; (3) compensatory changes, where the key positional amino acid residues to compensate for CDR transplantation; (4) positional conservation, where the humanized monoclonal antibody is humanized using the FR conserved sequence as a template but retaining key amino acid residues in the variable region of the non-humanized monoclonal antibody.

Included herein are not only intact monoclonal antibodies, but also immunologically active antibody fragments (antigen-binding fragments) such as Fab, Fab', F(ab')₂, Fd, single-chain Fv or scFv, disulfide-bonded Fv, V-NAR structural domains, IgNar, intracellular antibodies, IgGΔCH2, mini-antibodies, F(ab')₃, tetra-antibodies, triple-antibodies, bispecific antibodies, single domain antibodies, DVD-Ig, Fcab, mAb2, (scFv)2 or scFv-Fc,etc.

In some embodiments, the antibodies disclosed herein do not competitively bind TIGIT with Tiragolumab. In some embodiments, the antibody binding epitopes disclosed herein are different from those of Tiragolumab. In some embodiments, the above-mentioned antibodies are, for example, 7103-06, or 7103-07.

In some embodiments, the antibody disclosed herein competitively binds to TIGIT with Tiragolumab and partially blocks Tiragolumab binding. In some embodiments, the antibody binding epitopes disclosed herein overlap with Tiragolumab. In some embodiments, the above-mentioned antibodies are, for example, 7103-01, 7103-03, or 7103-04.

In some embodiments, the antibodies disclosed herein bind to the same epitope. In some embodiments, antibodies 7103-01, 7103-03, and 7103-04 block each other's TIGIT binding and bind to the same epitope.

In some embodiments, the antibodies disclosed herein bind to TIGIT of humans or other primates, but do not bind to TIGIT of mice.

Monoclonal antibodies and fragments thereof can be prepared using various methods familiar to those skilled in the art. For example, monoclonal antibodies can be prepared using the hybridoma method (first proposed by Kohler et al., Nature, 256:495 (1975)), or using the recombinant DNA method (US Patent No. 4816567). Monoclonal antibodies may also be isolated from phage antibody libraries by techniques described, for example, in Clackson et al, Nature, 352:624-628 (1991) and Marks et al, J. Mol. Biol., 222:581-597 (1991).

### Encoding molecules of monoclonal antibodies or fragments thereof, expression vectors containing the molecule, and host cells

Also provided herein are nucleic acid molecules encoding the anti-TIGIT monoclonal antibodies or fragments thereof. The sequences of these nucleic acid molecules can be obtained by conventional techniques, for example, methods such as PCR amplification or genomic library screening. In addition, the sequences encoding light chain and heavy chain can be fused together, to form a single-chain antibody. Also disclosed herein are expression vectors containing the aforementioned nucleic acid molecules, for example, pcDNA3.4, pcDNA3.1, and the like; and host cells transformed by the aforementioned expression vectors, which may be, for example, CHO cells, COS cells, and the like, are also disclosed herein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

It has been possible now to obtain a nucleic acid sequence encoding the monoclonal antibody (or a fragment thereof, or a derivative thereof) according to the present invention completely by chemical synthesis. The sequence can then be introduced into various existing molecules (or, for example, vectors) and cells known in the art. In addition, mutations can be introduced into the protein sequence of the present invention by chemical synthesis.

The present invention also relates to a vector comprising the encoding sequence as described above and an appropriate promoter or control sequence. These vectors can be used to transform appropriate host cells to enable them to express proteins. Host cells may be prokaryotic cells, such as bacterial cells; or lower eukaryotic cells, such as yeast cells; or higher eukaryotic cells, such as mammalian cells, such as CHO cells, COS cells, etc. Mammalian cell lines are typically used as host cells for expressing eukaryotic derived peptides. The proliferation of mammalian cells in culture is well-known in this field. See "Tissue Culture", Academic Press, Kruse and Patterns Editing (1973), which is incorporated herein by reference. Preferred mammalian cells are a number of commercially available infinitely proliferating cell lines. These infinitely proliferating cell lines include, but are not limited to, Chinese hamster ovary (CHO) cells, Vero cells, Hela cells, baby hamster kidney (BHK) cells, monkey kidney cells (COS), human hepatocellular carcinoma cells (such as Hep G2), and many other cell lines. They provide post-translational modifications for protein molecules, including correct folding, correct formation of disulfide bonds, and glycosylation at the correct sites.

Also provided herein are hybridoma cell lines that can produce monoclonal antibodies herein. After obtaining the hybridoma producing the monoclonal antibody herein, a person skilled in the art can conveniently know the structure of the antibody herein (such as the heavy chain variable region and the light chain variable region of the antibody), and then prepare the monoclonal antibody herein.

There are many methods for transforming host cells using expression vectors, and the transformation program used depends on the host to be transformed. The method of introducing heterologous polynucleotides into mammalian cells is known in the art, which includes glucan mediated transfection, calcium phosphate precipitation, Polybrene (1,5-dimethyl-1,5-diazaundecamethyiene polymethobromide) mediated transfection, protoplast fusion, electroporation, liposome mediated transfection, and direct microinjection of DNA into the nucleus. In the present invention, the preferred methods are electroporation or liposome-mediated methods or the like. For example, Invitrogen's liposome assay kit can be used to transfect host cells such as COS and CHO cells.

Under conditions suitable for expression of the monoclonal antibody according to the present invention, the host cell obtained is cultured. Then, the monoclonal antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

### Identification and purification of monoclonal antibodies

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The monoclonal antibody according to the present invention can be expressed in a cell or on the cell membrane, or is secreted extracellularly. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting out method), centrifugation, osmotic bacteria disruption, ultrasonic treatment, ultracentrifugation, molecular sieve chromatography (gel filtration), adsorption chromatography, ion exchange chromatography, high performance liquid chromatography (HPLC), various other liquid chromatographic techniques, and combinations of these methods.

### Conjugates, chimeric antigen receptors, and immune cells expressing chimeric antigen receptors

Also provided in the present application are conjugates containing antibodies or antigen binding fragments thereof, chimeric antigen receptors, and immune cells expressing the chimeric antigen receptors. Due to the specificity of the antibody or antigen binding fragment thereof to TIGIT, the conjugate, chimeric antigen receptor, and immune cell expressing the chimeric antigen receptor can target cells or tissues expressing or overexpressing TIGIT. Moreover, due to the inherent biological activity of the antibody or antigen binding fragment thereof in the present application (such as immunomodulatory activity), the antibody or antigen binding fragment moiety in its conjugate, chimeric antigen receptor, and immune cells expressing chimeric antigen receptor can also play corresponding roles.

In some embodiments, the antibody conjugate may include: the antibody or antigen binding fragment thereof as described herein; coupling moieties, such as drugs, toxins, cytokines, radionuclides or enzymes; and optional, linkers. In some embodiments, the coupling is achieved through enzymatic coupling, chemical coupling, fusion, and other methods. The linker can be a "cleavable linker" that facilitates the release of coupling moieties in cells, such as an acid labile linker, a peptidase sensitive linker, a photolabile linker, a dimethyl linker, and a disulfide-containing linker.

In some embodiments, the coupling moiety linked to the antibody may be, for example, a bioactive agent, which can be any synthetic, recombinant, or naturally occurring substance that enhances and/or regulates the desired biological action. In some embodiments, the effects that the bioactive agent may provide include but are not limited to: regulating, stimulating, and/or inhibiting growth signals; regulating, stimulating, and/or inhibiting anti apoptotic signals; regulating, stimulating, and/or inhibiting apoptosis or necrosis signals; regulating, stimulating, and/or inhibiting the ADCC cascade; regulating, stimulating, and/or inhibiting the CDC cascade, etc. For example, it can be a chemotherapy agent, toxin, cytokine, isotope, enzyme, etc.

The present application also provides a chimeric antigen receptor (CAR) comprising an extracellular domain and an intracellular domain, wherein the extracellular domain comprises the antibody or antigen binding fragment thereof of the present application. In some embodiments, the CAR of the present application also includes a transmembrane domain connecting the extracellular domain and the intracellular domain, such as the transmembrane moiety of CD28. The CAR of the present application can be the first, second, third, or fourth generation CAR.

In some embodiments, the antibody or antigen binding fragment thereof in the CAR of the present application is scFv or VH sdAb, or composed thereof. In some embodiments, the intracellular domains of CAR include those that simulate or approximate signals through natural antigen receptors, signals through the combination of such receptors and co-stimulatory receptors, and/or signals through individual co-stimulatory receptors.

In some embodiments, the intracellular domain includes one or more co-stimulatory domains and activation domains, such as one or more selected from the following group consisting of: ITAM domain, CD3ζ, CD28, 4-1BB, OX40, CD27, ICOS or a combination thereof, such as (CD28 +CD3ζ), (CD28 +CD27 + CD3ζ), (CD28 + OX40 + CD3ζ), (CD28 + 4-1BB + CD3ζ), (CD28 + CD27 + OX40 + CD3ζ), (CD28 + 4-1BB + CD27 + CD3ζ), (CD28 + 4-1BB + OX40 + CD3ζ), (4-1BB + CD3ζ), (4-1BB + OX40 + CD3ζ), (4-1BB + CD27 +CD3ζ), (CD27 + CD3ζ), (CD27 + OX40 + CD3ζ), (CD28Δ +CD3ζ), (CD28Δ +CD27 + CD3ζ), (CD28Δ + OX40 + CD3ζ), (CD28Δ + 4-1BB + CD3ζ), (CD28Δ + 4-1BB + OX40 + CD3ζ), (CD28Δ + CD27 + OX40 + CD3ζ), (CD28Δ + 4-1BB + CD27 + CD3ζ)), (4-1BB + ICOS + CD3ζ), (CD28 + ICOS + CD3ζ), (ICOS + CD3ζ).

In some embodiments, the extracellular domain of CAR further comprises a hinge region, for example, the hinge region is derived from an IgG hinge or a CD8α/CD28 extracellular region, such as selected from: IgG4 Fc Δ EQ, IgG4 Fc Δ Q, (t-12AA + t-20AA), mKate, phiLov, dsRed, Venus, eGFP, CH3 HA, (CD8 α + t-20AA), double T-20 AA, (t-20 AA + CD8α), (CD8α + leucine zipper Basep1), (CD8α+ leucine zipper Acid1), 2D3, CD8α, or IgG4Fc.

The present application also provides a transformed immune cell, which expresses the chimeric antigen receptor of the present application. For example, the immune cell is selected from T cells, such as αβ T cells, γδ T cells or NK T cells or T cells derived from pluripotent cells. Immune cells can be obtained from individuals to be treated or from other sources. Transformed (optionally amplified) immune cells can be administered to the individual to be treated, for example, for adoptive therapy.

In addition, the present application also includes CAR constructs, expression vectors, preparation methods, and their applications for transforming immune cells.

### Pharmaceutical composition

Also provided herein is a pharmaceutical composition comprising a pharmaceutically effective amount of a monoclonal antibody herein or an immunoconjugate thereof and a pharmaceutically acceptable carrier.

As used herein, the term "pharmaceutically acceptable" refers to the absence of adverse, allergic, or other adverse reactions when the molecular body and composition are appropriately administered to animals or humans. As used herein, the term "pharmaceutically acceptable carrier" should be compatible with the active substance herein, i.e., can be blended with it without significantly reducing the effectiveness of the drug composition under normal circumstances. These carriers are well known to those of ordinary skill in the art. And detailed discussion about the pharmaceutically acceptable carrier can be found in Remington's Pharmaceutical Sciences (Mack Pub. Co., N.J., 1991).

Such carriers include, but are not limited to, saline, buffer solution, glucose, water, glycerin, ethanol, adjuvant or the combination thereof. Further, these carriers can contain auxiliary substance(s), such as wetting agent or emulsifying agent, pH buffering substance, etc.

The composition herein can be administered orally, intravenously, intramuscularly, or subcutaneously; preferably oral or intravenous injection. The pharmaceutical composition herein can be made into various dosage forms according to needs, and the physician can determine the beneficial dosage for patients based on factors such as patient type, age, weight, approximate disease condition, and administration method.

When a pharmaceutical composition is used, a safe and effective amount of the immune conjugate is administered to a mammal, wherein the safe and effective amount is typically about 0.1-5 µg/kg body weight, and in most cases no more than about 3 mg/kg body weight, preferably about 1-10 µg/kg body weight to about 1 mg/kg body weight. Of course, the specific dosage should also consider factors such as the administration route and the patient's health status, which are all within the skill range of a skilled physician.

As used herein, the term "unit dosage form" refers to the dosage form required for single administration of the disclosed composition, including but not limited to various solid agents (such as tablets), liquid agents, capsules, and sustained-release agents, for the convenience of administration.

In another preferred embodiment in the present disclosure, the composition is a unit dosage form or multiple dosage forms, and the content of the active substance is 0.01-2000mg/dose, preferably 0.1-1500mg/dose, and more preferably 1-1000mg/dose. In another preferred embodiment in the present disclosure, 1-6 doses of the composition of the present disclosure are administered daily, preferably from 1 to 3; most preferably, 1 dose per day.

The monoclonal antibody or immune conjugate in the present disclosure can effectively block the binding of TIGIT and its ligand CD155, enhance the release of cytokines of immune cells, improve the activity of immune cells, and enhance immune response, which can be used to prevent and/or treat tumors, infections or infectious diseases. The active substance herein can be used to treat various types of tumors, such as (but not limited to) adenocarcinoma, leukemia, lymphoma, melanoma, sarcoma; tumor tissues from the adrenal gland, gallbladder, bone, bone marrow, brain, breast, bile duct, gastrointestinal tract, heart, kidney, liver, lungs, muscles, ovaries, pancreas, parathyroid gland, penis, prostate, skin, salivary gland, spleen, testes, thymus, thyroid or uterus; tumors of the central nervous system, such as glioblastoma or astrocytoma; eye tumors (such as basal cell carcinoma, squamous cell carcinoma, or melanoma), endocrine gland tumors, neuroendocrine system tumors, gastrointestinal pancreatic endocrine system tumors, reproductive system tumors, or head and neck tumors.

### Detection kit

Also provided herein is a kit for detecting TIGIT, comprising an anti-TIGIT monoclonal antibody or an active fragment thereof as described herein, an immunoconjugate. The kit herein can be used to detect whether TIGIT or content thereof is present in a biological sample. The detection method comprising the steps of: (a) contacting a sample with an anti-TIGIT monoclonal antibody or an immunoconjugate thereof in a kit herein; (b) detecting whether an antigen-antibody complex is formed, wherein the complex is formed to indicate the presence of TIGIT or quantitative detection of the amount of antigen-antibody complex formed in the sample to reflect the content of TIGIT in the sample. The sample can be pretreated or not pretreated, for example, may be extracted, purified, or concentrated.

The kit comprises a container and a monoclonal antibody herein located within the container, or a detection plate containing the monoclonal antibody, as well as a user manual. The kit may also comprise other reagents required for detection, such as buffer, indicator, etc. A person skilled in the art may adjust the contents of the kit according to specific needs.

### Examples

The present disclosure is further explained below in conjunction with specific example. It should be understood that these examples are only for illustrating the present disclosure and not intend to limit the scope of the present disclosure. Those skilled in the art may make appropriate modifications and variations to the present disclosure, which are within the scope of the present disclosure.

Experimental methods that do not indicate specific conditions in the following examples may employ conventional methods in the art, such as reference to "Molecular Cloning Experiment Guide " (Third Edition, New York, Cold Spring Harbor Laboratory Press, New York: Cold Spring Harbor Laboratory Press, 1989) or according to the conditions suggested by the supplier. The sequencing method of DNA is a conventional method in the art, and may also be tested by commercial companies.

Unless otherwise stated, percentages and parts are calculated by weight. Unless otherwise defined, all professional and scientific terms used herein have the same meaning as those familiar to those skilled in the art. In addition, any method and material similar or equivalent to what is described may be applied to the method of the present disclosure. The preferred embodiments and materials described herein are exemplary only.

### Example 1. Generation of Anti-Human TIGIT Mouse Monoclonal Antibody

### 1.1 immunization

50 µg of recombinant human TIGIT (Human TIGIT His, Sino Biological, 10917-H08H Assessment # NP 776160.2. Met1-Phe138, with expressed multi-histidine tag at C-terminus) was emulsified in the presence of a complete Freund's adjuvant (CFA) according to 100 µl of protein and 100 µl of CFA and subjected to multi-point immunization (each injection quantity is 50 µl) via abdominal and subcutaneous to 3 BALB/c mice (6-8 week-aged, weighing around 18g, female, purchased from the Experimental Animal Management Department of the Institute of Family Planning Science). Emulsification was also carried out in the presence of incomplete Freund 's adjuvant as well as 100 µl of protein and 100 µl IFA. Two weeks, three weeks, four weeks and five weeks after the first immunization, mice were boosted with the same immunogen hTIGIT for four times. After another week, the serum titer was detected to reach more than 100000, and the same 25 µg of immunogen hTIGIT was used to boost via subcutaneous injection for myeloma cell fusion.

### 1.2 Fusion

Spleen cells and lymphocytes of mice were fused with mouse myeloma cells (purchased from Cell Bank of Chinese Academy of Sciences, Item No. TCM 18)., The specific steps were as follows:
The myeloma cells (passaged before 24 hours) of 2 T75 FLasks were taken washed once with an IMDM medium, and resuspended and counted as 2-4×10⁷ cells. After 72 hours of last boost immunization, the eyeballs of mice were bled, necked and euthanized, and disinfected in 75% ethanol. Spleen was removed under sterile condition, surrounding connective tissue was stripped, and washed with IMDM medium. The spleen cells were cut and ground, blown away, filtered through a 40 um cell filter screen, centrifuged at 1000 rpm, 5 min, resuspended, and counted as 1-2×10⁸. The two kinds of cells were evenly mixed and centrifuged at 1000 rpm for 5 min. The cell supernatant was discarded, and the centrifuge tube bottom was tapped by hand, so that the precipitate was loose and preheated in a 37°C water bath. 1 mL of PEG preheated to 37°C was added dropwise to the centrifuge tube at 60s, and gently stirred for about 1 min while adding. 1 ml IMDM medium was added within 30s, then 3 ml IMDM medium was added within 1 min, and finally 16 ml IMDM medium was added within 1 min. The mixture was placed for 10 min, centrifuged at 1000 rpm for 5 min. The supernatant was discarded, cells were resuspended with an IMDM medium containing 20% FBS (containing 1 x Hat (Sigma, H0262-10 VL) and 1 x antibiotic solution (double antibody) (Gibco, 15140122)), subpackaged in 25 cell culture 96-well plates (200 µL/well,) and incubated at 37°C with 5% CO₂.

### 1.3 Hybridoma Screening

After fusion for 5-10 days, according to the cell growth density, the whole medium was changed, and the culture medium was the IMDM medium containing 10% FBS (containing 1 x HAT and 1 x double antibody), 200 µl/well, incubated at 37°C with 5% CO₂. After fusion for 10-14 days, ELISA detection was performed according to cell growth density. The detected positive cells was subcloned for the first time and the second time. The ELISA detection step was as follows:
TIGIT-Fc (Human TIGIT (Fc Tag): Sino Biological, 10917- H02 H) coating: the TIGIT-Fc was diluted into a concentration of 1 µg/mL with a coating solution, mixed well and then sucked into a 96-well ELISA plate with a porous pipettor (50 µl/well), sealed with a sealing film and placed overnight in a refrigerator at 4 °C.

The ELISA plate blocking: the plate was washed twice with PBS (250 µl/well), and patched dry. Then 150 µl/well blocking solution was added, and the plate was sealed with a sealing film and placed at 37°C for 1.5 hours.

Primary antibody incubation: the hybridoma was taken and added into the ELISA plate (100 µl/well), and the plate was sealed with a sealing film and placed at 37°C for 1 hour.

Secondary antibody incubation: the blocking solution was discarded. The plate was washed with 250 µl/well PBST for 3 times, and patched dry. The goat anti-mouse HRP secondary antibody (Jackson Immuno Research, 115-035-164) was diluted with the binding solution at 1: 3000, added into the ELISA plate at 100 µl/well, and the plate was sealed with a sealing film and placed at 37°C for 1 hour.

TMB incubation: the blocking solution was discarded. The plate was washed with 250 µl of PBST for 3 times, and patched dry. TMB (50 ul/well) was added into the ELISA plate, and the plate was sealed with a sealing film and placed at 37°C for 15-25 minutes.

Microplate Reader Detection: dual wavelength 450nm/655nm detection. The results were calculated as OD₄₅₀- OD₆₅₅.

### 1.4 Preparation of mouse monoclonal antibodies

A total of 8 clones were screened, and their information was shown in Table 1:

**Table 1. Clone Information**

| **No.** | **Clone** | **Heavy chain** | **Light chain** |
|---|---|---|---|
| 7103-01 | 31E10-2-1 | IgG1 | kappa |
| 7103-02 | 37C6-1-1 | IgG1 | kappa |
| 7103-03 | 36H8-2-2 | IgG1 | kappa |
| 7103-04 | 40E6-2-2 | IgG1 | kappa |
| 7103-05 | 41D7-2-1 | IgG1 | kappa |
| 7103-06 | 47A2-1-2 | IgG1 | kappa |
| 7103-07 | 42C12-1-2 | IgG1 | kappa |
| 7103-08 | 49F8-1-2 | IgG2b | kappa |

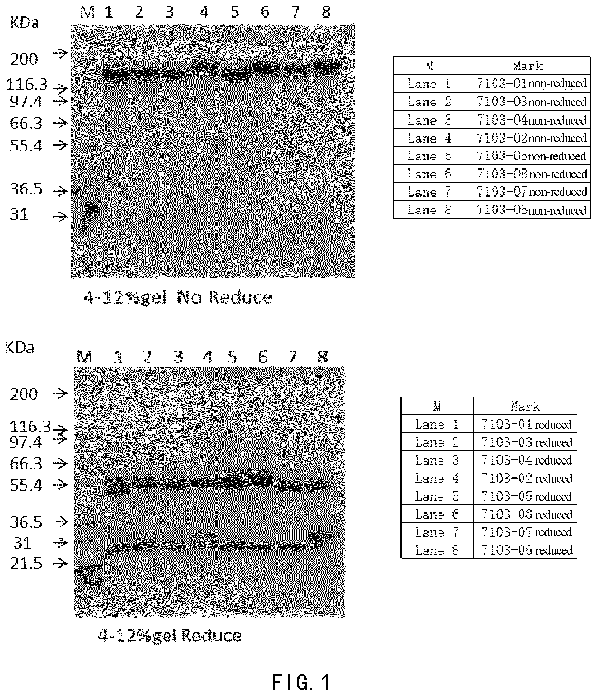

The resultant cell strains were cultured and cells were amplified. The cell culture solution was centrifuged, the supernatant was collected, and the fusion protein was purified from the supernatant by a Protein-A affinity chromatography column. The protein was analyzed by running glue, and 2 µg of reduced and non-reduced samples were loaded on each lane respectively (Voltage 150 V, 1 hour). The molecular weight of the non-reduced antibody is about 150 kD, and the molecular weight of the reduced antibody is about 50 kD and about 25 kD, respectively, which conforms to the molecular weight characteristics of the antibody (Fig. 1).

### 1.5 Identification of Mouse Monoclonal Antibody

### 1.5.1 Binding of anti-human TIGIT mouse monoclonal antibody to recombinant human TIGIT antigen

By ELISA, the binding of eight TIGIT monoclonal antibodies with recombinant human TIGIT antigen (Human TIGIT (His), Sino Biological, 10917- H08H) was tested using standard methods and steps as follows :
TIGIT coating: the TIGIT was diluted into a concentration of 1 µg/mL with a coating solution, mixed well and then sucked into a 96-well ELISA plate with a porous pipettor(50 µl/well), sealed with a sealing film and placed overnight in a refrigerator at 4 °C.

The ELISA plate blocking: the plate was washed twice with PBS (250 µl/well), and patched dry. Then 150 µl/well blocking solution was added, and the plate was sealed with a sealing film and placed at 37°C for 1.5 hours.

Primary antibody incubation: antibody was serially diluted, added into the ELISA plate (100 µl/well), and the plate was sealed with a sealing film and placed at 37°C for 1 hour.

Secondary antibody incubation: the blocking solution was discarded. The plate was washed with 250 µl/well PBST for 3 times, and patched dry. The goat anti-mouse HRP secondary antibody (Jackson Immuno Research, 115-035-164) was diluted with the binding solution at 1: 3000, added into the ELISA plate at 100 µl/well, and the plate was sealed with a sealing film and placed at 37°C for 1 hour.

TMB incubation: the blocking solution was discarded, the plate was washed with 250 µl of PBST for 3 times, and patched dry. TMB (50 ul/well) was added into the ELISA plate, and the plate was sealed with a sealing film and placed at 37°C for 15-25 minutes.

Microplate Reader Detection: dual wavelength 450nm/655nm detection. The results were calculated as OD₄₅₀- OD₆₅₅.

### Experimental Results and analysis

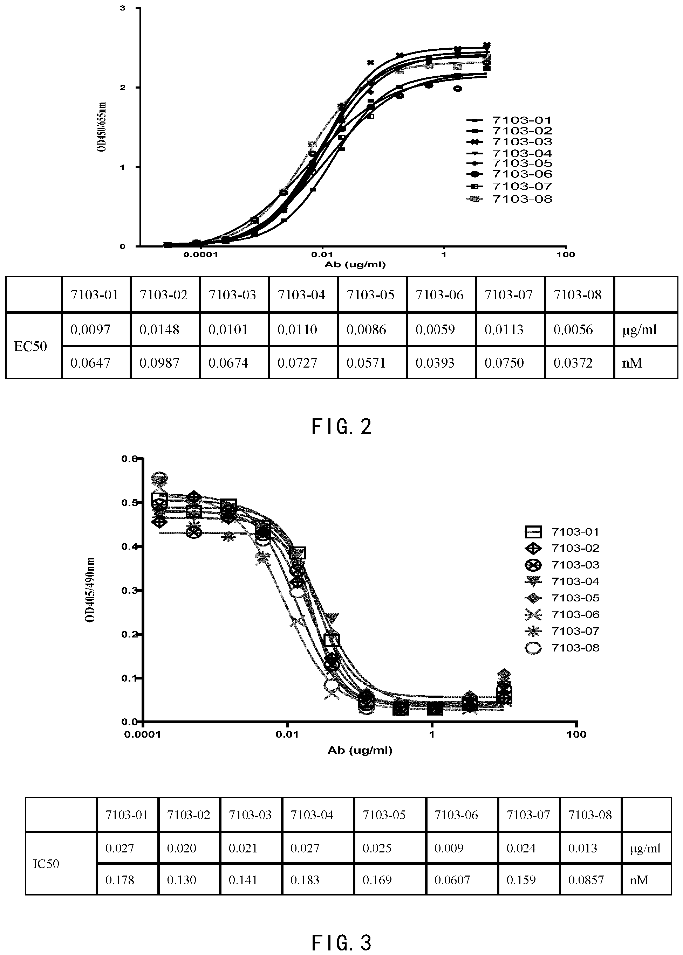

The ELISA test results showed (Fig. 2) that 8 anti-human TIGIT mouse monoclonal antibodies (7103-01 to7103-08) shown in Table 1 could specifically bind to human TIGIT, and the binding affinity EC 50 was 0.0647 nM, 0.0987 nM, 0.0674 nM, 0.0727 nM, 0.0571 nM, 0.0393 nM, 0.075 nM, and 0.0372 nM, respectively.

### 1.5.2 Inhibition of Recombinant Human TIGIT-CD155 Binding by Anti-Human TIGIT Mouse Monoclonal Antibody

In order to evaluate the competition between the anti-human TIGIT mouse monoclonal antibody and CD155 (Human CD155 (Fc Tag): Sino Biological, 10109- H02H) for binding to recombinant human TIGIT (Human TIGIT(His), Sino Biological, 10917- H08H), ELISA detection was performed, and the specific steps were as follows:
TIGIT coating: the TIGIT was diluted into a concentration of 0.8 µg/mL with a coating solution, mixed well and then sucked into a 96-well ELISA plate with a porous pipettor (50 µl/well), sealed with a sealing film and placed overnight in a refrigerator at 4 °C.

The ELISA plate blocking: the plate was washed twice with PBS (250 µl/well), and patched dry. Then 150 µl/well blocking solution was added, and the plate was sealed with a sealing film and placed at 37°C for 1.5 hours.

Primary antibody incubation: the plate was washed twice with PBS (250 µl/well), and patched dry. 6 µg/ml of CD155 was respectively diluted in a binding solution and added into a 96-well dilution plate at 60 µl/well. Then the screened hybridoma antibody was diluted with the binding solution, (the highest concentration was 30 µg/ml), and diluted to 3 times of concentration, and added into the corresponding dilution plate above at 60 µl/well. After mixing well, the sample was added to the coated 96-well ELISA plate at 100 µl/well. The plate was sealed with a sealing film and placed at 37°C for 1.5 hours.

Secondary antibody incubation: the blocking solution was discarded, the plate was washed with 250 µl PBST for 3 times, and patched dry. Anti-Human-Fc-AP (Jackson Immuno Research, 109-055-098) was diluted with the binding solution 1: 3000, added into the ELISA plate at 100 µl/well, and the plate was sealed with a sealing film and placed at 37°C for 1 hour.

PNPP incubation: the blocking solution was discarded, the plate was washed with 250 µl PBST for 3 times, and patched dry. One PNPP (SouthernBiotech, 0201-01) was taken and added into 1 mL 5 x diethanolamine substrate, then 4 mL of deionized water was added for dissolution, and the sample was added into ELISA plate at 50 µL/well, and the plate was sealed with a seal film and placed at 37°C for 15-25 minutes.

Microplate Reader Detection: dual wavelength 405nm/490nm detection. The results were calculated with OD₄₀₅- OD₄₉₀.

### Experimental results and analysis

The experimental results (Fig. 3) showed that the eight anti-human TIGIT mouse monoclonal antibodies shown in Table 1 could specifically block the binding of CD155 and human hTIGIT, and IC₅₀ were 0.178 nM, 0.130 nM, 0.141 nM, 0.183 nM, 0.169 nM, 0.0607 nM, 0.159 nM, and 0.0857 nM, respectively.

### 1.5.3 Binding of anti-human TIGIT mouse monoclonal antibody to recombinant cynomolgus monkey TIGIT

The cynomolgus monkey or mouse TIGIT antigen of was coated with the same method as above, the anti-human TIGIT mouse monoclonal antibody was diluted in gradient, and then the anti-mouse Fab secondary antibody was added (Jackson Immuno Research, 515-035-072) for TMB development. ELISA was used to detect the binding of 8 mouse monoclonal antibodies to recombinant cynomolgus monkey TIGIT.

### Experimental results and analysis

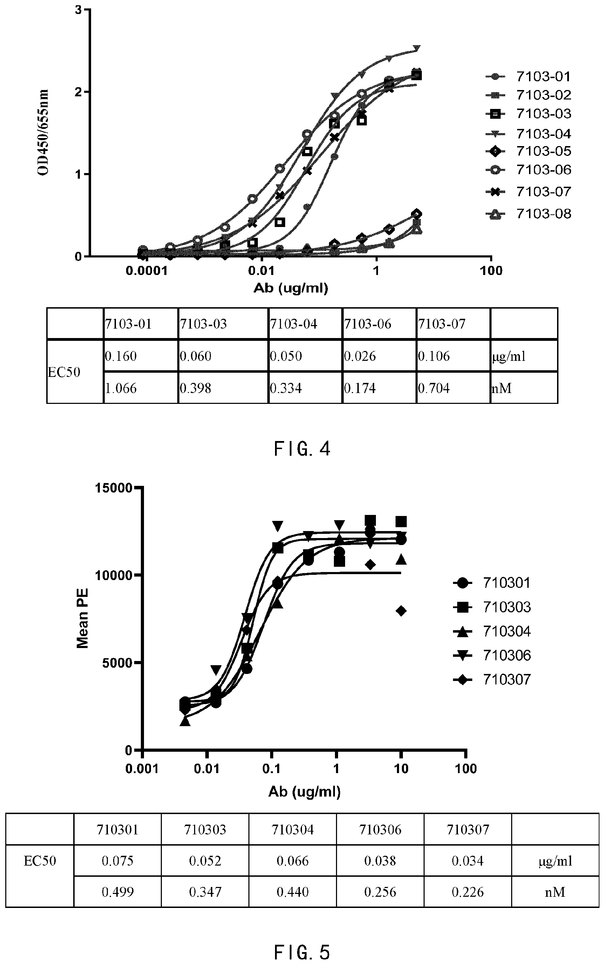

The result is shown in Fig. 4, the eight anti-human TIGIT mouse monoclonal antibodies all bind to human TIGIT and none binds to the murine TIGIT.

In the eight strains, five strains of the anti-human TIGIT mouse monoclonal antibody (numbered 7103-01, 7103-03, 7103-04, 7105-06, 7103-07) could recognize the cynomolgus monkey TIGIT, and the binding EC50 was 1.066 nM, 0.398 nM, 0.334 nM, 0.174 nM, and 0.704 nM, respectively. However, 3 strains of anti-human TIGIT mouse monoclonal antibodies (numbered 7103-02, 7103-05, 7103-08) do not bind to cynomolgus monkey TIGIT.

### 1.5.4. Binding of anti-human TIGIT mouse monoclonal antibody to TIGIT expressed on CHO cell membrane

The above-mentioned purified anti-human TIGIT mouse monoclonal antibodies that could bind with the cynomolgus monkey TIGIT were used to bind with TIGIT on the cell membrane, and the antibody binding strength was analyzed by the flow cytometry analysis technology. The specific steps were as follows:
The CHO-K (CHO-K-TIGIT) cell strain stably expressing full-length human TIGIT was constructed as follows: the cDNA was cloned into a mammalian cell expression vector pcDNA3.1 (Invitrogen) containing a rat glutamine synthetase gene by using a plasmid (purchased from Sino Biological) containing full-length human TIGIT cDNA (NM_173799.2), then the constructed plasmid was transfected into CHO-K cells by using an electro-transfection (Bio-Rad, Gene Pulser Xcell) method. The transfected cells were cultured in an OptiCHO medium (Invitrogen) for 24-48 hours, and the culture medium was replaced into a screening medium. The screening medium contained OptiCHO, 5 µg/ml recombinant human insulin and 10 µM aminosulfoxide methionine (MSX). Cells were cultured in incubator at 37°C, 8% CO₂. After 3 weeks, the screened cultured cells were subjected to flow cytometry (FACS) with an anti-TIGIT antibody, thereby obtaining a CHO-K monoclonal cell strain with human TIGIT expressed on the cell membrane.

The CHO-TIGIT was counted and centrifuged at 1000 rpm for 5 min, resuspended with PBS containing 0.5% BSA and were added into a 96-well sharp-bottom plane at 5×10⁵ cells/well. After centrifuged at 400g for 5 min, the supernatant was discarded. The anti-TIGIT antibody was serially diluted and added to a 96-well sharp-bottom plane at 100 µl/well, placed at 4°C for 30 min. The plate was washed with PBS containing 0.5% BSA at 250 µl/well twice. 1: 100 diluted Anti-Mouse-IgY-PE (Jackson Immuno Research, 115-116-071) was added into the 96-well sharp-bottom plate at 100 µl/well, and placed at 4°C for 30 min. The plate was washed with PBS containing 0.5% BSA at 250 µl/well twice. PBS containing 0.5% BSA was added at 200 µl/well. The samples were detected with Beckman CytoFLEX.

### Experimental results and analysis

Results were shown in Fig. 5, each of five anti-human TIGIT mouse monoclonal antibodies (7103-01, 7103-03, 7103-04, 7105-06, 7103-07)could coubind with TIGIT (CHO-TIGIT) expressed on the surface of the cell membrane, and the EC₅₀ for binding the TIGIT on CHO cell membrane was 0.499 nM, 0.347 nM, 0.440 nM, 0.256 nM, and 0.226 nM, respectively.

### 1.6 Sequencing of Heavy and Light Chain Variable Regions

### 1.6.1. RNA Extraction and cDNA Acquisition

About 1-2×10⁵ hybridoma cells were taken for RNA extraction (Sigma, GenElute^{™} Mammalian Total RNA Miniprep Kit, Item No. RTN70). About 8 µl of total RNA was taken for subsequent reverse transcription PCR: first 65°C, 5 min to eliminate the secondary structure of RNA; followed by adding 2 µl of 5 x gDNA wiper, 42°C, 2 min to remove genomic DNA contamination. Finally, 2 µL of 10 x RT Mix, 2 µL of HiscriptIII Enzyme Mix, and 1 µL of Random Hexamers (Novozyme, R312) were added. The walls of the tubes were flicked for well mixing, the liquid was thrown to the bottom of the tubes by brief centrifugation, and finally the reverse transcription reaction was carried out by using the following conditions: 25°C, 5 min; 42°C, 45 min; and 85°C, 5s.

### 1.6.2. Amplification of antibody variable region gene

The above cDNA was used as a template, and the VH and VL genes of the hybridoma antibody were amplified by a PCR method with different primer combinations. The primers used in the experiment mainly refer to the document von Boehmer, L. et al., Sequencing and cloning of antigen-specific antibodies from mouse memory B cells. Nat Protoc, 2016. 11(10): p. 1908-1923. The heavy chain was obtained with two different sets of primers primerHa (including SEQ ID NO: 1 to SEQ ID NO: 12) and primerHb (SEQ ID NO: 13 and SEQ ID NO: 14), and the light chain was obtained with primerKb (including SEQ ID NO: 15 to SEQ ID NO: 24) and primerKc (SEQ ID NO: 25 and SEQ ID NO: 26), respectively. The reaction conditions were as follows: denaturation at 94°C for 3 min followed by 40 cycles, each at 94°C, 30s, 55°C, 30s, and 72°C, 35s, and incubation at the end of the cycle at 72 °C for 5 minutes.

**Table 2. Primers for amplifying antibody variable region genes**

| **Description** | | **SEQ ID NO** | **sequence** |
|---|---|---|---|
| Primer of heavy chain | Primer rHa | 1 | AGGAACTGCAGGTGTCC |
| | | 2 | CAGCTACAGGTGTCCACTCC |
| | | 3 | TGGCAGCARCAGCTACAGG |
| | | 4 | CTGCCTGGTGACATTCCCA |
| | | 5 | CCAAGCTGTGTCCTGTC |
| | | 6 | TTTTAAAAGGTGTCCAGKGT |
| | | 7 | CCTGTCAGTAACTRCAGGTGTCC |
| | | 8 | TTTTAAAAGGGGTCCAGTGT |
| | | 9 | CGTTCCTGGTATCCTGTCT |
| | | 10 | ATGAAGTTGTGGYTRAACTGG |
| | | 11 | TGTTGGGGCTKAAGTGGG |
| | | 12 | AGAAGGTGTGCACACCGCTGGAC |
| | Primer rHb | 13 | GGGAATTCGAGGTGCAGCTGCAGGAGTCTGG |
| | | 14 | GCTCAGGGAARTAGCCCTTGAC |
| Primer of light chain | Primer rKb | 15 | RGTGCAGATTTTCAGCTTCCTGCT |
| | | 16 | TGGACATGAGGGCYCCTGCTCAGT |
| | | 17 | CTSTGGTTGTCTGGTGTTGAYGGA |
| | | 18 | GTTGCTGCTGCTGTGGCTTACA |
| | | 19 | GTATCTGGTACCTGTGG |
| | | 20 | TGCCTGTTAGGCTGTTGGTGCT |
| | | 21 | GCTCAGTTCCTTGGTCTCCTGTTGC |
| | | 22 | CAGTTCCTGTTTCTGTTARTGCTCTGG |
| | | 23 | TGCTCTGGTTATATGGTGCTGATGGG |
| | | 24 | ACTGAGGCACCTCCAGATGTT |
| | Primer rKc | 25 | GAYATTGTGMTSACMCARWCTMCA |
| | | 26 | TGGGAAGATGGATACAGTT |

The obtained PCR products were either directly sequenced or sequenced via TA clone, and the sequencing primer for the heavy chain was SEQ ID NO: 14, and the sequencing primer for the light chain was SEQ ID NO: 26. The V-region sequences of all the antibodies were successfully obtained via IgBlast comparison, as shown in the following table:

**Table.3 V-region sequences of 8 strains of TIGIT mouse monoclonal antibodies**

| **No.** | **Description** | | **SEQ ID NO** | **Sequence** |
|---|---|---|---|---|
| 7103-01 | VH | Full-length sequence | 27 | QVQLQQPGAELVKPGASVKLSCKASGYTFIS YNIYWVKQRPGQGLEWIGGVNPSNGNTNF NENFKSKATLTVDKSSSTAYMQLSSLTSDDS AVYFCTRGNYYGYEFAYWGQGTLVTVSA |
| | | CDR1 | 28 | SYNIY |
| | | CDR2 | 29 | GVNPSNGNTNFNENFKS |
| | | CDR3 | 30 | GNYYGYEFAY |
| | VL | Full-length sequence | 31 | DIVMTQSHKFMSTSVGDRVSITCKASQHVST AVAWYQQKPGQSPKLLIYSPSYRYTGVPDR FTGSGSGTDFTFTISSVQAEDLAVYYCQQHY STPWTFGGGTKLEIK |
| | | CDR1 | 32 | KASQHVSTAVA |
| | | CDR2 | 33 | SPSYRYT |
| | | CDR3 | 34 | QQHYSTPWT |
| 7103-02 | VH | Full-length sequence | 35 | EVQLQQSGPELVKPGASVKMSCKASGYTITS YVMHWVKQKPGQGLEWIGYINPYNDGTKY NEKFKGKATLTSDKSSSTAYMELSSLTSEDS AVYYCARWNPYYYAMDYWGQGTSVTVSS |
| | | CDR1 | 36 | SYVMH |
| | | CDR2 | 37 | YINPYNDGTKYNEKFKG |
| | | CDR3 | 38 | WNPYYYAMDY |
| | VL | Full-length sequence | 39 | EVVLTQSPAITAASLGQKVTITCSANSSARYV HWYQQKSGTSPKPWIYEISKLASGVPARFSG SGSGTSYSLTISSMGAEDAAIYYCQQWNYPL ITFGAGTKLEIK |
| | | CDR1 | 40 | SANSSARYVH |
| | | CDR2 | 41 | EISKLAS |
| | | CDR3 | 42 | QQWNYPLIT |
| 7103-03 | VH | Full-length sequence | 43 | EVQLQQSGPELVKPGASVKISCKTSGYTFTE YTMHWVKQSHGKSLEWIGGINPNNGGTSY NQKFKGKATLTVDKSSSTAYMELRSLTSED SAVYYCARGAYYRYDFDYWGQGTTLTVSS |
| | | CDR1 | 44 | EYTMH |
| | | CDR2 | 45 | GINPNNGGTSYNQKFKG |
| | | CDR3 | 46 | GAYYRYDFDY |
| | VL | Full-length sequence | 47 | DIVMTQSHKFMSTSVGDRVSITCKASQDVR TAVVWYQQKPGQSPKLLIYSASYRYTGVPD RFTGSGSGTDFTFTISSVQAEDLAVYYCQQH YSTPWTFGGGTKL E I K |
| | | CDR1 | 48 | KASQDVRTAVV |
| | | CDR2 | 49 | SASYRYT |
| | | CDR3 | 50 | QQHYSTPWT |
| 7103-04 | VH | Full-length sequence | 51 | VQLQESGAELVKPGASVKLSCRASGYTFTDY FVYWVKQRPGQGLEWIGGINPSNGGATFNE KFKNKATLTVDTSSSTAYMQLSSLTSEDSAI YYCTRGNFYGYEFAYWGQGTLVTVSA |
| | | CDR1 | 52 | DYFVY |
| | | CDR2 | 53 | GINPSNGGATFNEKFKN |
| | | CDR3 | 54 | GNFYGYEFAY |
| | VL | Full-length sequence | 55 | DIVMTQSHKFISTSVGDRVSITCKASQHVST AVVWYQQKPGQSPKLLIYSPSYRYTGVPDR FTGSGSGTDFTFTISSVQAEDLAVYYCQQHY ITPWTFGGG TKLEIK |
| | | CDR1 | 56 | KASQHVSTAVV |
| | | CDR2 | 57 | SPSYRYT |
| | | CDR3 | 58 | QQHYITPWT |
| 7103-05 | VH | Full-length sequence | 59 | EVQLQQSGPALVKPGASVKISCKTSEYTFTE YTMYWVKQSHGKSLEWIGGINPNNGGTRY NQKFKGKATLTVDKSSSTAYMELRSLTSED SAVYYCARGGHYDYGFAYWGQGTLVTVSA |
| | | CDR1 | 60 | EYTMY |
| | | CDR2 | 61 | GINPNNGGTRYNQKFKG |
| | | CDR3 | 62 | GGHYDYGFAY |
| | VL | Full-length sequence | 63 | DIVMTQSHKFMSTSVGDRVSITCKASQDVK TAVAWYQQKPGQSPKVLIYSPSYRYTGVPD RFTGSGSGTDFTFTISSVQAEDLAVYYCQQH YSTPWTFGGGTKLKSK |
| | | CDR1 | 64 | KASQDVKTAVA |
| | | CDR2 | 65 | SPSYRYT |
| | | CDR3 | 66 | QQHYSTPWT |
| 7103-06 | VH | Full-length sequence | 67 | EEQLVESGGGLVKPGGTLKLSCAASGFTFST YAMSWVRQSPEKRLEWVAEISSGGSYTYYS DTVTGRFTISRDNAKNTLYLEMSSLRSEDTA MYYCARKTLDYYALDYWGHGTSVTVSS |
| | | CDR1 | 68 | TYAMS |
| | | CDR2 | 69 | EISSGGSYTYYSDTVTG |
| | | CDR3 | 70 | KTLDYYALDY |
| | VL | Full-length sequence | 71 | DVQITQSPSYLAASPGETITINCRANKTISKY LAWYQEKPGKTNKLLIYSGSRLQSGIPSKFS GSGYGTDFTLTISSLEPEDFAMYYCQQHNEY PWTFGGGTKLEIK |
| | | CDR1 | 72 | RANKTISKYLA |
| | | CDR2 | 73 | SGSRLQS |
| | | CDR3 | 74 | QQHNEYPWT |
| 7103-07 | VH | Full-length sequence | 75 | EVQVVESGGGLVKPGGTLKLSCAASGFTFST YAMSWVRQSPEQRLEWVAEISSGGSHTFYS DTVTGRFTISRDNAQNTLYLEMNSLRSEDTA MYYCARKTLDYYALDYWGQGTSVTVSS |
| | | CDR1 | 76 | TYAMS |
| | | CDR2 | 77 | EISSGGSHTFYSDTVTG |
| | | CDR3 | 78 | KTLDYYALDY |
| | VL | Full-length sequence | 79 | DVQITQSPSYLAASPGETITINCRASKSISKYL AWYQEKPGKTNKLLIYSGSRLQSGIPSKFSG SGYGTDFTLTISSLEPEDFAMYYCQQHNEYP WTFGGGTKVEIK |
| | | CDR1 | 80 | RASKSISKYLA |
| | | CDR2 | 81 | SGSRLQS |
| | | CDR3 | 82 | QQHNEYPWT |
| 7103-08 | VH | Full-length sequence | 83 | EVQLQESGPELVKPGASVKISCKASGYSFTG YYMLWVKQSHVKSLEWIGRINPYNGATTY NQNFKDKASLTVNKSSSTAYMELHSLTSEDS AVYYCARWFGDFDFWGQGTTLTVSS |
| | | CDR1 | 84 | GYYML, |
| | | CDR2 | 85 | RINPYNGATTYNQNFKD |
| | | CDR3 | 86 | WFGDFDF |
| | VL | Full-length sequence | 87 | DIQMTQSPSSLSASLGERVSLTCRASQEISGY LSWLQQKPDGTIKRLIYATSTLDSGVPKRFS GSRSGSDYSLTISSLESEDFADYYCLQYASYP FTFGSGTKLEIK |
| | | CDR1 | 88 | RASQEISGYLS |
| | | CDR2 | 89 | ATSTLDS |
| | | CDR3 | 90 | LQYASYPFT |

| | | | | |
|---|---|---|---|---|
| The underline represents CDR. | | | | |

### Example 2. Preparation of Human Mouse Chimeric Monoclonal Antibody

### 2.1 Construction of Expression Plasmid for Human Mouse Chimeric Monoclonal Antibody

In order to perform the recombinant expression of the antibody, the V-region of the hybridoma antibody was constructed on the chimeric antibody expression vector (pcDNA3.1), i.e., the V-region sequence was connected to the expression vector whose constant region was human IgG1 or human λ/κ.. Firstly, specific PCR primers were designed according to different sequences, and the sequences were shown in the following table. PCR reaction conditions were as follows: after denaturation at 94°C for 3 minutes, 40 cycles at 94°C, 30 seconds, 55°C, 30 seconds, 72°C, 35 seconds, incubation at 72°C for 5 minutes. The fragment for cloning was successfully retrieved by using a PCR product with a definite sequencing result or a TA clone bacterial solution as a template.

**Table 4. Primer Sequence for Chimeric Antibody Expression Vector Construction**

| No. | SEQ ID NO | Specific sequence |
|---|---|---|
| 7103-01-L-F | 91 | GCTTCCCAGGCGCCAGATGCGACATTGTGATGACMCAGTC |
| 7103-01-L-R | 92 | ATGGAGCGGCCACAGTACGTTTKATTTCCARCTTKGTSCC |
| 7103-02-L-F | 93 | GCTTCCCAGGCGCCAGATGCGAAAWTGTGCTCACCCAGTC |
| 7103-02-L-R | 94 | ATGGAGCGGCCACAGTACGTTTCAGCTCCAGCTTGGTCCC |
| 7103-03-L-F | 95 | GCTTCCCAGGCGCCAGATGCGACATTGTGATGACMCAGTC |
| 7103-03-L-R | 96 | ATGGAGCGGCCACAGTACGTTTKATTTCCARCTTKGTSCC |
| 7103-04-L-F | 97 | GCTTCCCAGGCGCCAGATGCGACATTGTGATGACMCAGTC |
| 7103-04-L-R | 98 | ATGGAGCGGCCACAGTACGTTTKATTTCCARCTTKGTSCC |
| 7103-05-L-F | 99 | GCTTCCCAGGCGCCAGATGCGACATTGTGATGACMCAGTC |
| 7103-05-L-F2 | 100 | GCTTCCCAGGCGCCAGATGCGACATTGTGATGACACAGTC |
| 7103-05-L-R | 101 | ATGGAGCGGCCACAGTACGTTTKATTTCCARCTTKGTSCC |
| 7103-06-L-F | 102 | GCTTCCCAGGCGCCAGATGCGATGTCCAGATAACCCAGTC |
| 7103-06-L-R | 103 | ATGGAGCGGCCACAGTACGTTTKATTTCCARCTTKGTSCC |
| 7103-07-L-F | 104 | GCTTCCCAGGCGCCAGATGCGATGTCCAGATAACCCAGTC |
| 7103-07-L-R | 105 | ATGGAGCGGCCACAGTACGTTTKATTTCCARCTTKGTSCC |
| 7103-08-L-F | 106 | GCTTCCCAGGCGCCAGATGCGACATCCAGATGACHCAGTC |
| 7103-08-L-R | 107 | ATGGAGCGGCCACAGTACGTTTKATTTCCARCTTKGTSCC |
| 7103-01-H-F | 108 | TAAAAGGTGTCCAGTGTCAGGTCCAACTGCAGCAGCC |
| 7103-01-H-R | 109 | GGCCCCTTTGTTGATGCTGCAGAGACAGTGACCAGAG |
| 7103-02-H-F | 110 | TAAAAGGTGTCCAGTGTSAGGTYCAGCTGCARCAGTC |
| 7103-02-H-R | 111 | GGCCCCTTTGTTGATGCTGAGGAGACGGTGACTGAGG |
| 7103-03-H-F | 112 | TAAAAGGTGTCCAGTGTSAGGTYCAGCTGCARCAGTC |
| 7103-03-H-R | 113 | GGCCCCTTTGTTGATGCTGAGGAGACTGTGAGAGTGG |
| 7103-04-H-F | 114 | TAAAAGGTGTCCAGTGTSAGGTYCAGCTGCARCAGTC |
| 7103-04-H-R | 115 | GGCCCCTTTGTTGATGCTGCAGAGACAGTGACCAGAG |
| 7103-05-H-F | 116 | TAAAAGGTGTCCAGTGTSAGGTYCAGCTGCARCAGTC |
| 7103-05-H-R | 117 | GGCCCCTTTGTTGATGCTGCAGAGACAGTGACCAGAG |
| 7103-06-H-F | 118 | TAAAAGGTGTCCAGTGTGAWGTGCAGCTGGTGGAGTC |
| 7103-06-H-R | 119 | GGCCCCTTTGTTGATGCTGAGGAGACGGTGACTGAGG |
| 7103-07-H-F | 120 | TAAAAGGTGTCCAGTGTGAWGTGCAGCTGGTGGAGTC |
| 7103-07-H-R | 121 | GGCCCCTTTGTTGATGCTGAGGAGACGGTGACTGAGG |
| 7103-08-H-F | 122 | TAAAAGGTGTCCAGTGTSAGGTYCAGCTGCARCAGTC |
| 7103-08-H-R | 123 | GGCCCCTTTGTTGATGCTGAGGAGACTGTGAGAGTGG |

The PCR product was used for agarose gel electrophoresis, the target stripe was recovered, XHO I single-enzyme digestion reaction was performed, the enzyme-cut PCR fragment was cloned into the expression vector of the same enzyme digestion, the recombinant plasmid was transformed into DH5a competent bacteria, the positive colonies containing correct recombinant plasmids were identified by a colony PCR method, and recombinant plasmids, enzyme digestion and sequencing identification were purified.

### 2.2 Expression of Human Mouse Chimeric Monoclonal Antibody

HEK293F cells, culture medium and transfection reagent used in the experiment were all purchased from Sino Biological, Inc., and were used as follows: one day before transfection, samples were taken to calculate the cell density and viability, and inoculated into fresh culture medium at a density of 2×10⁶ cells/mL, and placed in a constant-temperature shaking incubator at 37°C, 5% CO₂, and 150-175 rpm for culture. On the day of transfection, the cell viability was calculated and the cells were adjusted to 3 ×10⁶ cells/mL. Preparation of the transfection dye: 20ml transfection volume was taken as a example, 20 µg of DNA was diluted with 150 mM NaCl, and the total volume was 500 µL, and the mixture was gently mixed; 100 µL of sinofection transfection reagent was diluted with 150 mM NaCl, and the total volume was 500 µL, and mixed gently; the diluted DNA and transfection reagent were placed at the same time for 5 min, then mixed gently and uniformly, wherein the total volume of the diluted DNA and transfection reagent was 1 ml, then placed for 10 min, and finally dropwise added into the cell culture solution, and cultured continuously after well mixing. After the 3rd day after transfection, the viability of the cells was detected every day, and the supernatant was harvested when the cell viability was about 60%.

### 2.3 Purification of Human Mouse Chimeric Monoclonal Antibody

The above chimeric monoclonal antibodies (numbered 7103-01 (hFc), 7103-03 (hFc), 7103-04 (hFc), 7103-06(hFc), 7103-07(hFc)) were expressed by mammalian cells and purified, the cell culture solution harvested after transient transfection was centrifuged at 4000 rpm for 10 min, and the cells were discarded. An appropriate amount of protein A beads were added to the supernatant, and incubated at room temperature for 4 h. After the supernatant was removed by centrifugation, the protein A beads were washed twice with PBS, and finally eluted with a citric acid eluent with pH3, and finally the pH was adjusted to be neutral with 1 M Tris.

### Experimental results and analysis

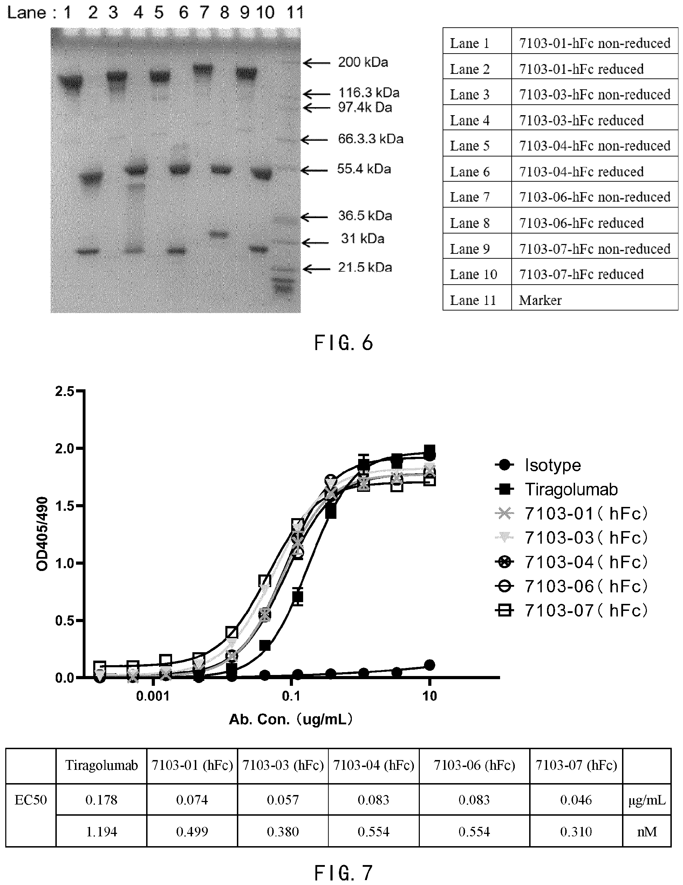

The results of the SDS-PAGE analysis after purification were shown in Fig. 6. The molecular weight of the non-reduced antibody was about 150 kDa, and the molecular weight of the reduced antibody was about 55 kDa and about 25 kDa, respectively, which conforms to the molecular weight characteristics of the antibody.

### Example 3. Binding of Human Mouse Chimeric Monoclonal Antibody to TIGIT

### 3.1 The in vitro binding activity of human mouse chimeric monoclonal TIGIT antibody and hTIGIT was studied by ELISA method

In order to determine whether the human mouse chimeric TIGIT monoclonal antibody of the present disclosure could bind to the recombinant human TIGIT protein, *in vitro* testing was performed. Tiragolumab was used as a positive control.

Antigen hTIGIT (Human TIGIT (His), Sino Biological, 10917- H08H) was diluted into 1 µg/mL with a NaHCO₃ solution of pH 9.6, 50 µl per well was added to a 96-well ELISA plate, and placed overnight in refrigerator at 4°C. The next day, after washing twice with PBS, blocked with 3% BSA, 100 µL per well, incubated at 37°C for 1.5 h, washed 4 times with PBST, primary antibody diluted was added and incubated continuously at 37°C for 2h, and then washed 4 times with PBST, a certain proportion of diluted anti-human-Fc-AP antibody (Jackson Immuno Research, 109-055-098) was added, incubated at 37°C for 1.5 h, and finally washed 4 times with PBST. TMB substrate (p-nitrophenyl phosphate, SouthBiotech 109-055-09) was added, incubated at 37°C for 10-15 min, and the readings were obtained with the microplate reader (at wavelength of 405 nm and 490 nm).

### Experimental results and analysis

The ELISA test results (Fig. 7) showed that 5 types of anti-TIGIT human mouse chimeric monoclonal antibodies (numbered 7103-01 (hFc), 7103-03 (hFc), 7103-04 (hFc), 7103-06 (hFc), 7103-07 (hFc)) could specifically bind to human TIGIT, and the binding affinity EC50 thereof was 0.499 nM, 0.380 nM, 0.554 nM, 0.554 nM and 0.310 nM, respectively, and EC50 of positive control Tiragolumab was 1.194 nM.

The result showed that the recombinant expressed chimeric antibody binding antigen TIGIT had good activity and was equivalent to a murine antibody purified by hybridoma. Compared with the Tiragolumab of Gentech, the antibody of the present disclosure has better binding activity for TIGIT, and EC50 values were only 1/4-1/2 of that of Tiragolumab.

### 3.2 Research on the Binding Activity of Human Mouse Chimeric TIGIT Monoclonal Antibody with TIGIT Using Biofilm Layer Optical Interference Technology (BLI)

The dissociation constant (Kd) of the human mouse chimeric TIGIT monoclonal antibody was detected by using a GATORS (ProbeLife) detection instrument and an HFC (LOT# 2007065 Tray 2), Human antibody Capture probe. The human mouse chimeric TIGIT monoclonal antibody was diluted to 30nM in a binding buffer (Q buffer [PBS (10mM PH7.4) + 0.02% Tween 20+0.2% BSA]). The recombinant human TIGIT protein was diluted in Q buffer in a 2-fold gradient to different concentrations varying in the range of 40 nM to 10 nM. A kinetic correlation determination was initiated by placing an antibody capture sensor in the continuously diluted antigen solution described above. Octet Data Acquisition software was opened and New Kinetics Experiment-Basic Kinetics mode was selected.

Process was set up according to the following table:

**Table 5. Process for studying TIGIT antibody binding activity using biofilm layer optical interferometry (BLI)**

| Process | Time | Concentrate |
|---|---|---|
| Baseline 1 | 30s | Q buffer |
| Loading | 120s | 30 nM antibody ( in Q buffer) |
| Baseline 1 | 60s | Q buffer |
| Relevance | 120s | 40nM-10nM, antigens diluted with a 2-fold gradient of Q buffer |
| Dissociation | 120s | Q buffer |
| Regeneration | 25s | Q buffer |

**Table 6. Dynamics analysis results of human mouse chimeric monoclonal antibodies for binding with human TIGIT**

| **Sample ID** | **TIGIT-His** | | | | |
|---|---|---|---|---|---|
| | **K_{D} (M)** | **ka (1/Ms)** | **kd (1/s)** | **R²** | **Rₘₐₓ (nm)** |
| 7103-01 (hFc) | 5.74E-10 | 7.06E+05 | 4.05E-04 | 0.992 | 0.209 |
| 7103-03 (hFc) | 1.14E-09 | 8.01E+05 | 9.10E-04 | 0.992 | 0.237 |
| 7103-04 (hFc) | 6.61E-10 | 8.45E+05 | 5.58E-04 | 0.989 | 0.213 |
| 7103-06 (hFc) | 1.29E-09 | 1.07E+06 | 1.39E-03 | 0.983 | 0.221 |
| 7103-07 (hFc) | 1.72E-09 | 9.71E+05 | 1.67E-03 | 0.986 | 0.229 |

Experimental results showed that the correlation coefficient R² of all the antibodies in the Global fitting mode was greater than 0.95, which met system adaptability requirements and was reliable in result.

### 3.3 Binding of Human Mouse Chimeric Monoclonal Antibody to TIGIT Protein on Cell Membrane Using Flow Cytometry Method

In order to determine whether the mouse chimeric TIGIT monoclonal antibody of the present disclosure could bind to TIGIT protein on the cell membrane, *in vitro* testing was performed. Tiragolumab was used as a positive control.

The FACS buffer was first formulated, i.e., 1 x PBS +0.5% BSA solution. A required number of cells were taken, the amount of cells required for one sample was about 1×10⁵-5×10⁵. The primary antibody was diluted in a certain proportion using FACS buffer. The cells were resuspended (the volume was about 100 µL), incubated at 4°C for 30 min in a refrigerator, and after the cells were washed once with FACS buffer, FITC-anti-human IgG (Abcam: 6854) diluted with FACS buffer was used to resuspend the cells and then the cells were placed in a refrigerator at 4°C for incubation for 30 min. Finally, after washing the cells with the FACS buffer, the cells wer resuspended with 200 µL of FACS buffer, and detected on a machine.

### Experimental results and analysis

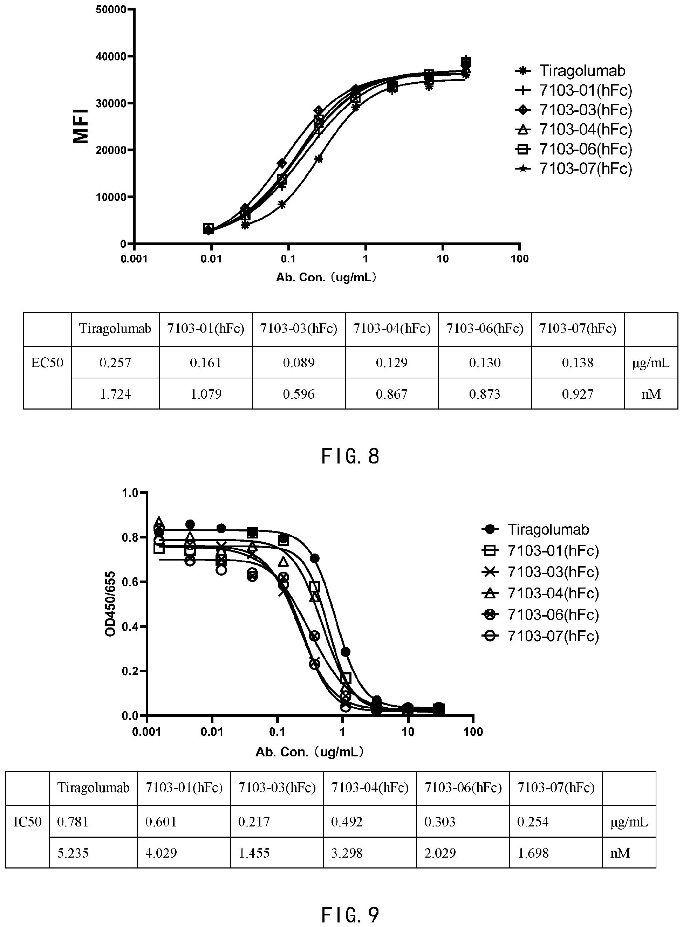

Results were shown in Fig. 8. Five TIGIT chimeric monoclonal antibodies (numbered 7103-01 (hFc), 7103-03 (hFc), 7103-04 (hFc), 7103-06 (hFc), 7103-07 (hFc)) could well bind with CHO-TIGIT, and EC50 on the CHO-TIGIT cell membrane was 1.079 nM, 0.596 nM, 0.867 nM, 0.873 nM, and 0.927 nM, respectively while the positive control Tiragolumab was 1.724 nM.

The data indicated that the antibodies of the present disclosure not only had a strong binding capacity with antigen on a solid-phase (ELISA method), but also had a very high binding activity to TIGIT on a cell membrane, and EC50 values were 1/3-1/2 of Tiragolumab, indicating that the binding of the antibodies of the present invention to TIGIT on the cell membrane was significantly better than the binding of Tiragolumab to TIGIT on the cell membrane surface.

### Example 4. Inhibition of Recombinant Human TIGIT-CD155 Binding by Human Mouse Chimeric TIGIT Monoclonal Antibody

In order to determine whether the human mouse chimeric TIGIT monoclonal antibody of the present disclosure could inhibit the binding of CD155 to TIGIT, *in vitro* testing was performed. Tiragolumab was used as a positive control.

Antigen TIGIT (Human TIGIT (His), Sino Biological, 10917- H08H) was diluted with pH 9.6 of NaHCO₃ solution into 1 µg/mL, 50 µL per well was added to a 96-well ELISA plate, and placed overnight at 4°C in a refrigerator. The next day, after washing with PBS twice, plate was blocked with 3% BSA (100 µL per well) by incubating at 37°C for 1.5 h, and washed 4 times with PBST. Gradient-diluted primary antibody and CD155-mFC (6µg/mL) (Human CD155(Fc Tag): Sino Biological, 10109-H02H) were mixed in equal volumes and added to a blocked and washed enzyme-linked immunosorbent assay (ELISA) plate; incubated at 37°C for 2 h; followed by another 4 washes with PBST; a certain proportion of diluted secondary antibody HRP anti-mFC (Jackson Immuno Research, 115-035-164) was added, incubated at 37°C for 1.5h; and finally, after 4 washes with PBST, TMB substrate was added, incubated at 37°C for 10-15 min, and the readings were obtained with microplate reader (at wavelength of 450 nm and 655 nm).

### Experimental results and analysis

Experimental results (Fig. 9) showed that five chimeric TIGIT monoclonal antibodies (numbered 7103-01 (hFc), 7103-03 (hFc), 7103-04 (hFc), 7103-06 (hFc), 7103-07 (hFc)) were capable of specifically blocking the binding of CD155 to human HTIGIT. IC₅₀ was 4.029 nM, 1.455 nM, 3.298 nM, 2.029 nM, and 1.698 nM, respectively while IC₅₀ of positive control Tiragolumab was 5.235 nM.

It can be obtained from the above data that the antibody of the present disclosure can block binding of TIGIT and ligand CD155 thereof, and the antibody blocking capability of the present disclosure is better than that of Tiragolumab, wherein the IC₅₀ values of 7103-03 (hFc), 7103-06 (hFc), and 7103-07 (hFc) are 1/3-1/2 of Tiragolumab, so that the blocking capability is significantly better than that of Tiragolumab.

### Example 5. Binding Epitope Analysis of Human Mouse Chimeric TIGIT Monoclonal Antibody

In order to determine whether the binding epitope of the mouse chimeric TIGIT monoclonal antibody of the present disclosure with the disclosed Tiragolumab was crossed, an *in vitro* test was performed.

Antigen TIGIT (Human TIGIT (His), Sino Biological, 10917- H08H) was diluted with pH9.6 of NaHCO₃ solution into 1 µg/mL, 50 µL per well was added to a 96-well ELISA plate, and placed overnight at 4°C in a refrigerator. The next day, after two washes with PBS, the plate was blocked with 3% BSA (100 µL per well), incubated at 37°C for 1.5 h, and washed 4 times with PBST. Gradient-diluted murine antibody of the present disclosure and Tiragolumab (the final concentration was 0.83 µg/ml, which was equivalent to the EC₈₀ in combination with TIGIT-His) were mixed in equal volumes and added to a blocked and washed enzyme-linked immunosorbent assay (ELISA) plate; incubated at 37°C for 2 h; followed by 4 further washes with PBST; a certain proportion of diluted secondary antibody HRP anti-mFC (Jackson Immuno Research, 115-035-164) was added, incubated at 37°C for 1.5 h; and finally, after 4 washes with PBST, TMB substrate was added, incubated at 37°C for 10-15 min, and the microplate reader was readed (at wavelength of 450 nm and 655 nm ).

### Experimental results and analysis

The experimental results showed the basic situation of the TIGIT epitope binding between the antibody of the present disclosure and Tiragolumab. 7103-06, 7103-07 and Tiragolumab did not compete with each other, indicating that the binding epitope was different from the Tiragolumab. However, 7103-01, 7103-03, and 7103-04 partially blocked the binding of Tiragolumab and TIGIT, indicating that the binding epitopes of 7103-01, 7103-03, 7103-04 were overlapped, but with some differences. Next, the same method was used to detect the competitive binding of TIGIT between 7103-01, 7103-03, and 7103-04. The results showed that they blocked each other from binding to the antigen TIGIT, indicating that the binding epitopes of the three were consistent.

According to the binding epitope, the antibody of the present invention can be divided into two groups:
group A: antibodies 7103-06, 7103-07 that do not compete with Tiragolumab, and their binding epitopes are different from that of Tiragolumab; and
group B: antibodies 7103-01, 7103-03 and 7103-04 that partially block the binding of Tiragolumab to TIGIT have overlapping binding epitopes with Tiragolumab, but with some differences. Moreover, the antibodies 7103-01, 7103-03, 7103-04 block the TIGIT binding of each other, indicating that they bind to the same epitope.

### Example 6. The Generation of INFγ of PBMC was Enhanced in vitro by Human Mouse Chimeric TIGIT Monoclonal Antibody

After T cell activation, lymphocyte secretes cytokine IFN γ. In order to test the function of the human mouse chimeric TIGIT monoclonal antibody as a positive regulator for T cell activation, the following experiments were performed to demonstrate the enhancement of the antibody of the present disclosure on IFN γ production of PBMC cell via blocking TIGIT signal.

A commercially available frozen human peripheral blood mononuclear cell PBMC (Oricells) was recovered, the cell density was adjusted to 5×10⁶/mL, the PHA was diluted with RPMI 1640 +10% FBS medium to a concentration of 6 µg/mL, then the antibody was diluted into 20 µg/mL by using the PHA diluent. 100 µl of PHA and antibody diluent were added to a 96-well plate, and finally 100 µl of cell suspension was added to a 96-well plate and gently mixed, and cultured in an incubator at 37°C and 5% CO₂ for 4 days. After 4 days, 100 µl supernatant was sucked up for detecting content of IFNγ.

### Experimental results and analysis

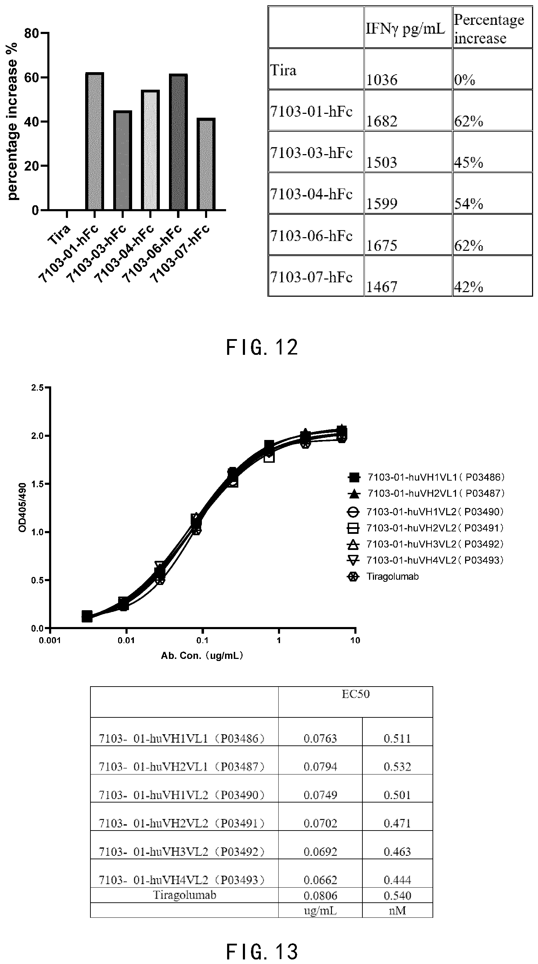

Fig. 12 showed that 5 human mouse chimeric TIGIT monoclonal antibodies (numbered 7103-01 (hFc), 7103-03 (hFc), 7103-04 (hFc), 7103-06 (hFc), 7103-07 (hFc), 7103-07 (hFc)) could promot PHA-activated PBMC to produce IFNγ, and the effectiveness was superior to that of Tiragolumab, with an increase in release percentage of 42%-62% as compared with Tiragolumab.

### Example 7. Design of Humanized Anti-Human TIGIT Monoclonal Antibody

According to the murine female parent antibody sequence numbered 7103-01 and 7103-07, a humanized antibody sequence was designed, and the specific steps were as follows: firstly, Discovery Studio and Schrödinger Antibody Modeling were used, and a homologous modeling method was used to construct a three-dimensional molecular model of the variable region. Next, the variable region and CDR of the female parent antibody were respectively subjected to structure simulation by comparing the existing antibody structure of the database. At the same time, a cDNA derived human germline sequence with high homology to murine female parent antibodies VH and VL, respectively, was selected for comparison. For heavy chain VH, IGHV1 with the highest homology was selected as the humanized design template to design the sequence. For light chain VL, IGKV1 was selected as the humanized design template to design the sequence.

### Results and Discussion

The murine female parent antibody number 7103-01 original heavy chain mVH sequence was designed into 4 humanized sequences: 7103-01-huVH1 (SEQ ID NO: 124), 7103-01-huVH2 (SEQ ID NO: 125), 7103-01-huVH3 (SEQ ID NO: 126) and 7103-01-huVH4 (SEQ ID NONO: 127); the original light chain mVL of the antibody was designed into two humanized sequences: 7103-01-huVL1 (SEQ ID NO:129) and 7103-01-huVL2 (SEQ ID NO:130).

The original heavy chain mVH sequence of the murine female parent antibody 7103-07 was designed into 5 humanized sequences: 7103-07-huVH1 (SEQ ID NO: 131), 7103-07-huVH2 (SEQ ID NO: 132), 7103-07-huVH3 (SEQ ID NO: 134), 7103-07-huVH4 (SEQ ID NO: 135), 7103-07-huVH5 (SEQ ID NO: 136); the original light chain mVL was designed into five humanized sequences: 7103-07-huVL1 (SEQ ID NO: 137), 7103-07-huVL2 (SEQ ID NO: 138), 7103-07-huVL3 (SEQ ID NO: 139), 7103-07-huVL4 (SEQ ID NO: 140), 7103-07-huVL5 (SEQ ID NO: 141).

The specific sequence are as follows:

**Table 7. V-Region Sequence of Humanized Antibody**

| No. | Description | | SEQ ID NO | Sequence |
|---|---|---|---|---|
| 7103-01 | huVH1 | Full-length sequence | 124 | QVQLVQSGAEVKKPGASVKLSCKASGYT FISYNIYWVRQAPGQGLEWIGGVNPSNG NTNFNENFKSRATLTVDKSSSTAYMELSR LRSDDTAVYFCTRGNYYGYEFAYWGQG TTVTVSS |
| | | CDR1 | 28 | SYNIY |
| | | CDR2 | 29 | GVNPSNGNTNFNENFKS |
| | | CDR3 | 30 | GNYYGYEFAY |
| | huVH2 | Full-length sequence | 125 | QVQLVQSGAEVKKPGASVKVSCKASGY TFISYNIYWVRQAPGQGLEWIGGVNPSN GNTNFNENFKSRVTLTVDKSISTAYMELS RLRSDDTAVYYCTRGNYYGYEFAYWGQ GTTVTVSS |
| | | CDR1 | 28 | SYNIY |
| | | CDR2 | 29 | GVNPSNGNTNFNENFKS |
| | | CDR3 | 30 | GNYYGYEFAY |
| | huVH3 | Full-length sequence | 126 | QVQLVQSGAEVKKPGASVKVSCKASGY TFISYNIYWVRQAPGQGLEWIGGVNPSN GNTNFNENFKGRVTLTVDTSISTAYMELS RLRSDDTAVYYCTRGNYYGYEFAYWGQ GTTVTVSS |
| | | CDR1 | 28 | SYNIY |
| | | CDR2 | 142 | GVNPSNGNTNFNENFKG |
| | | CDR3 | 30 | GNYYGYEFAY |
| | huVH4 | Full-length sequence | 127 | QVQLVQSGAEVKKPGASVKVSCKASGY TFISYNIYWVRQAPGQGLEWMGGVNPSN GNTNFNENFQGRVTMTVDTSISTAYMEL SRLRSDDTAVYYCTRGNYYGYEFAYWG QGTTVTVSS |
| | | CDR1 | 28 | SYNIY |
| | | CDR2 | 128 | GVNPSNGNTNFNENFQG |
| | | CDR3 | 30 | GNYYGYEFAY |
| | huVL1 | Full-length sequence | 129 | DIQMTQSPSSMSASVGDRVTITCKASQHV STAVAWYQQKPGKAPKLLIYSPSYRYTG VPSRFSGSGSGTDFTFTISSVQPEDIATYY CQQHYSTPWTFGGGTKLEIK |
| | | CDR1 | 32 | KASQHVSTAVA |
| | | CDR2 | 33 | SPSYRYT |
| | | CDR3 | 34 | QQHYSTPWT |
| | huVL2 | Full-length sequence | 130 | DIQMTQSPSSLSASVGDRVTITCKASQHV STAVAWYQQKPGKAPKLLIYSPSYRYTG VPSRFSGSGSGTDFTFTISSLQPEDIATYY CQQHYSTPWTFGGGTKLEIK |
| | | CDR1 | 32 | KASQHVSTAVA |
| | | CDR2 | 33 | SPSYRYT |
| | | CDR3 | 34 | QQHYSTPWT |
| 7103-07 | huVH1 | Full-length sequence | 131 | EVQVVESGGGLVKPGGSLRLSCAASGFT FSTYAMSWVRQAPGKGLEWVAEISSGGS HTFYSDTVTGRFTISRDNAKNTLYLQMN SLRAEDTAVYYCARKTLDYYALDYWGQ GTTVTVSS |
| | | CDR1 | 76 | TYAMS |
| | | CDR2 | 77 | EISSGGSHTFYSDTVTG |
| | | CDR3 | 78 | KTLDYYALDY |
| | huVH2 | Full-length sequence | 132 | EVQVVESGGGLVKPGGSLRLSCAASGFT FSTYAMSWVRQAPGKGLEWVAEISSGGS HTFYADTVKGRFTISRDNAKNTLYLQMN SLRAEDTAVYYCARKTLDYYALDYWGQ GTTVTVSS |
| | | CDR1 | 76 | TYAMS |
| | | CDR2 | 133 | EISSGGSHTFYADTVKG |
| | | CDR3 | 78 | KTLDYYALDY |
| | huVH3 | Full-length sequence | 134 | EVQLVESGGGLVKPGGSLRLSCAASGFTF STYAMSWVRQAPGKGLEWVAEISSGGSH TFYADTVTGRFTISRDNAKNTLYLQMNS LRAEDTAVYYCARKTLDYYALDYWGQG TTVTVSS |
| | | CDR1 | 76 | TYAMS |
| | | CDR2 | 143 | EISSGGSHTFYADTVTG |
| | | CDR3 | 78 | KTLDYYALDY |
| | huVH4 | Full-length sequence | 135 | EVQVVESGGGLVKPGGSLRLSCAASGFT FSTYAMSWVRQAPEQRLEWVAEISSGGS HTFYSDTVTGRFTISRDNAKNTLYLQMN SLRAEDTAVYYCARKTLDYYALDYWGQ GTTVTVSS |
| | | CDR1 | 76 | TYAMS |
| | | CDR2 | 77 | EISSGGSHTFYSDTVTG |
| | | CDR3 | 78 | KTLDYYALDY |
| | huVH5 | Full-length sequence | 136 | EVQVVESGGGLVKPGGSLRLSCAASGFT FSTYAMSWVRQAPEQRLEWVAEISSGGS HTFYADTVKGRFTISRDNAKNTLYLQMN SLRAEDTAVYYCARKTLDYYALDYWGQ GTTVTVSS |
| | | CDR1 | 76 | TYAMS |
| | | CDR2 | 133 | EISSGGSHTFYADTVKG |
| | | CDR3 | 78 | KTLDYYALDY |
| | huVL 1 | Full-length sequence | 137 | DVQITQSPSYLSASVGDRVTINCRASKSIS KYLAWYQQKPGKAPKLLIYSGSRLQSGIP SRFSGSGYGTDFTLTISSLQPEDFATYYCQ QHNEYPWTFGGGTKLEIK |
| | | CDR1 | 80 | RASKSISKYLA |
| | | CDR2 | 81 | SGSRLQS |
| | | CDR3 | 82 | QQHNEYPWT |
| | huVL2 | Full-length sequence | 138 | DVQITQSPSSVSASVGDRVTINCRASKSIS KYLAWYQQKPGKAPKLLIYSGSRLQSGV PSRFSGSGSGTDFTLTISSLQPEDFATYYC QQHNEYPWTFGGGTKLEIK |
| | | CDR1 | 80 | RASKSISKYLA |
| | | CDR2 | 81 | SGSRLQS |
| | | CDR3 | 82 | QQHNEYPWT |
| | huVL3 | Full-length sequence | 139 | DIQMTQSPSSVSASVGDRVTITCRASKSIS KYLAWYQQKPGKAPKLLIYSGSRLQSGV PSRFSGSGSGTDFTLTISSLQPEDFATYYC QQHNEYPWTFGGGTKLEIK |
| | | CDR1 | 80 | RASKSISKYLA |
| | | CDR2 | 81 | SGSRLQS |
| | | CDR3 | 82 | QQHNEYPWT |
| | huVL4 | Full-length sequence | 140 | DVQITQSPSSVSASVGETITINCRASKSISK YLAWYQQKPGKAPKLLIYSGSRLQSGVP SRFSGSGSGTDFTLTISSLQPEDFATYYCQ QHNEYPWTFGGGTKLEIK |
| | | CDR1 | 80 | RASKSISKYLA |
| | | CDR2 | 81 | SGSRLQS |
| | | CDR3 | 82 | QQHNEYPWT |
| | huVL5 | Full-length sequence | 141 | DIQMTQSPSSVSASVGETITITCRASKSISK YLAWYQQKPGKAPKLLIYSGSRLQSGVP SRFSGSGSGTDFTLTISSLQPEDFATYYCQ QHNEYPWTFGGGTKLEIK |
| | | CDR1 | 80 | RASKSISKYLA |
| | | CDR2 | 81 | SGSRLQS |
| | | CDR3 | 82 | QQHNEYPWT |

**Table 8. Combinations of different light and heavy chains of humanized antibodies and corresponding numbers**

| Source of humanized antibodies: | Combination of humanized antibody light and heavy chains | Combination number |
|---|---|---|
| 7103-01 | huVH1VL1 | P03486 |
| 7103-01 | huVH2VL1 | P03487 |
| 7103-01 | huVH3VL1 | P03488 |
| 7103-01 | huVH4VL1 | P03489 |
| 7103-01 | huVH1VL2 | P03490 |
| 7103-01 | huVH2VL2 | P03491 |
| 7103-01 | huVH3VL2 | P03492 |
| 7103-01 | huVH4VL2 | P03493 |
| 7103-07 | huVH1VL1 | P03476 |
| 7103-07 | huVH1VL2 | P03477 |
| 7103-07 | huVH1VL3 | P03478 |
| 7103-07 | huVH2VL1 | P03479 |
| 7103-07 | huVH2VL2 | P03480 |
| 7103-07 | huVH2VL3 | P03481 |
| 7103-07 | huVH3VL1 | P03482 |
| 7103-07 | huVH3VL2 | P03483 |
| 7103-07 | huVH3VL3 | P03484 |
| 7103-07 | huVH4VL2 | P04662 |
| 7103-07 | huVH5VL2 | P04663 |
| 7103-07 | huVH4VL5 | P04665 |
| 7103-07 | huVH5VL4 | P04666 |
| 7103-07 | huVH5VL5 | P04667 |

### Example 8. Identification of Humanized Anti-Human TIGIT Monoclonal Antibody

### 8.1 Construction of Expression Vector

According to the above humanized design result, the sequence was constructed onto the pcDNA3.4 vector, and the plasmid was obtained after PCR, enzyme digestion, linking, transformation, identification, sequencing, comparison and extraction.

### 8.2 Preparation of Various Combinations of Antibodies

According to the expression combination after the humanized design, the expression of 7 days in the ExpiCHO-S cell was performed by using the ExpiCHO-S expression system (Thermo Fisher, A29133) according to requirements, and the expression quantity was measured by HPLC on the 6th day. Finally, the antibody protein to be detected was obtained through one-step purification by Protein A affinity chromatography.

### 8.3 Binding of Humanized Monoclonal Antibody to TIGIT

### 8.3.1 Binding activity of humanized TIGIT monoclonal antibody to TIGIT was studied by Biofilm Layer Optical Interferometry (BLI)

The experimental method was the same as in Example 3.2.

The experimental results are shown in the following table :

**Table 9. Dynamics Analysis Results of Humanized Monoclonal Antibodies Binding to Human TIGIT**

| Table 9.1 Dynamics Analysis Results of Humanized Antibodies Designed with Murine Female Parent Antibody 7103-01 | | | | | | |
|---|---|---|---|---|---|---|
| **VH and VL combination** | **Sample ID** | **TIGIT-His** | | | | |
| | | **K_{D} (M)** | **ka (1/Ms)** | **kd (1/s)** | **R²** | **Rₘₐₓ (nm)** |
| 7103- 01 (hFc) | 7103- 01 (hFc) | 1.73E-09 | 5.49E+05 | 9.51E-04 | 0.992 | 0.293 |
| 7103-01-huVH1VL1 | P03486 | 1. 14E-09 | 5.11E+05 | 5.84E-04 | 0.991 | 0.292 |
| 7103-01-huVH2VL1 | P03487 | 1.43E-09 | 5.62E+05 | 8.04E-04 | 0.993 | 0.297 |
| 7103-01-huVH3VL1 | P03488 | 1.02E-09 | 5.11E+05 | 5.19E-04 | 0.993 | 0.301 |
| 7103-01-huVH4VL1 | P03489 | 1.19E-09 | 4.89E+05 | 5.84E-04 | 0.993 | 0.289 |
| 7103-01-huVH1VL2 | P03490 | 1.27E-09 | 5.22E+05 | 6.64E-04 | 0.993 | 0.303 |
| 7103-01-huVH2VL2 | P03491 | 1.88E-09 | 5.06E+05 | 9.50E-04 | 0.993 | 0.282 |
| 7103-01-huVH3VL2 | P03492 | 1.49E-09 | 5.25E+05 | 7.81E-04 | 0.993 | 0.291 |
| 7103-01-huVH4VL2 | P03493 | 1.12E-09 | 4.58E+05 | 5.13E-04 | 0.992 | 0.281 |

**Table 9.2 Dynamics Analysis Results of Humanized Antibodies Designed with Murine Female Parent Antibody 7103-07**

| **VH and VL combination** | **Sample ID** | **TIGIT-His** | | | | |
|---|---|---|---|---|---|---|
| | | **K_{D} (M)** | **ka (1/Ms)** | **kd (1/s)** | **R²** | **Rₘₐₓ (nm)** |
| 7103- 07 (hFc) | 7103-07 (hFc) | 2.96E-09 | 5.73E+05 | 1.69E-03 | 0.985 | 0.311 |
| 7103-07-huVH1VL1 | P03476 | 7.92E-09 | 6.10E+05 | 4.83E-03 | 0.992 | 0.264 |
| 7103-07-huVH1VL2 | P03477 | 5.95E-09 | 4.90E+05 | 2.91E-03 | 0.992 | 0.291 |
| 7103-07-huVH1VL3 | P03478 | 1.01E-08 | 7.07E+05 | 7.14E-03 | 0.993 | 0.236 |
| 7103-07-huVH2VL1 | P03479 | 8.43E-09 | 5.87E+05 | 4.95E-03 | 0.992 | 0.252 |
| 7103-07-huVH2VL2 | P03480 | 6.67E-09 | 6.05E+05 | 4.04E-03 | 0.986 | 0.223 |
| 7103-07-huVH2VL3 | P03481 | 1.00E-08 | 6.65E+05 | 6.66E-03 | 0.994 | 0.226 |
| 7103-07-huVH3VL1 | P03482 | 6.15E-09 | 6.61E+05 | 4.06E-03 | 0.99 | 0.23 |
| 7103-07-huVH3VL2 | P03483 | 7.77E-09 | 5.92E+05 | 4.60E-03 | 0.989 | 0.226 |
| 7103- 07-huVH3VL3 | P03484 | 9.15E-09 | 6.99E+05 | 6.39E-03 | 0.989 | 0.208 |
| 7103- 07-huVH4VL2 | P04662 | 2.40E-09 | 1.29E+06 | 3.10E-03 | 0.986 | 0.17 |
| 7103- 07-huVH5VL2 | P04663 | 2.00E-09 | 1.53E+06 | 3.07E-03 | 0.988 | 0.165 |
| 7103- 07-huVH4VL4 | P04664 | 2.73E-09 | 1.43E+06 | 3.91E-03 | 0.986 | 0.165 |
| 7103- 07-huVH4VL5 | P04665 | 5.58E-09 | 1.04E+06 | 5.83E-03 | 0.988 | 0.184 |
| 7103- 07-huVH5VL4 | P04666 | 2.19E-09 | 1.59E+06 | 3.47E-03 | 0.993 | 0.161 |
| 7103- 07-huVH5VL5 | P04667 | 4.20E-09 | 1.35E+06 | 5.65E-03 | 0.988 | 0.164 |

Experimental results showed that the correlation coefficient R² of all the antibodies in the Global fitting mode was greater than 0.95, which met system adaptability requirements and was reliable in result.

### 8.3.2 The in vitro binding activity of humanized monoclonal TIGIT antibody and HTIGIT was studied by ELISA method

In order to determine whether the humanized TIGIT monoclonal antibody of the present disclosure could bind to the recombinant human TIGIT protein, *in vitro* testing was performed. Murine antibodies and Tiragolumab (Tira) were used as positive controls. The experimental method was the same as in Example 3.1.

### Experimental results and analysis

ELISA test results (Fig. 13) showed that six strains of 7103-01-anti-human TIGIT humanized monoclonal antibodies (numbered P03486, P03487, P03490, P03491, P03492, P03493) could specifically bind to human TIGIT. The binding affinity EC₅₀ was 0.511 nM, 0.532 nM, 0.501 nM, 0.471 nM, 0.463 nM, and 0.444 nM, respectively. Compared with the 0.540nM of Tiragolumab, the humanized monoclonal antibody of the present invention had a higher affinity for binding to human TIGIT.

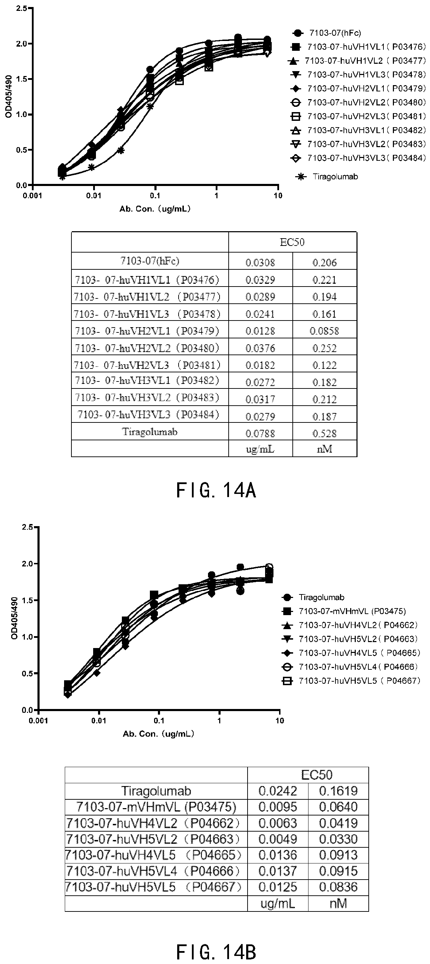

ELISA test results (Fig. 14A) showed that 9 strains of 7103-07 anti-human TIGIT humanized monoclonal antibodies (numbered P03476, P03477, P03478, P03479, P03480, P03481, P03482, P03483 and P03484) could specifically bind to human TIGIT. The binding affinity EC₅₀ was 0.221 nM, 0.194 nM, 0.161 nM, 0.0858 nM, 0.252 nM, 0.122 nM, 0.182 nM, 0.212 nM, and 0.187 nM, respectively, while EC₅₀ of human mouse chimeric antibody was 0.206 nM, and EC₅₀ of Tira was 0.528 nM, indicating that the recombinant expression humanized antibody had good activity in binding to the antigen TIGIT and was equivalent to human mouse chimeric antibody, and was superior to Tiragolumab (Tira).

The second batch of experiments were conducted using the same method, and the ELISA test results (Fig. 14B) showed that 5 strains of 7103-07 anti-human TIGIT humanized monoclonal antibodies (numbered P04662, P04663, P04664, P04665, P04666 and P04667) could specifically bind to human TIGIT. The binding affinity EC₅₀ was 0.0419 nM, 0.0330 nM, 0.0913 nM, 0.0915 nM, and 0.0836 nM, respectively, while EC₅₀ of human mouse chimeric antibody was 0.0640 nM, and the EC₅₀ of Tira was 0.1619 nM, indicating that the recombinant expressed humanized antibody had good activity in binding to the antigen TIGIT, and was equivalent to human mouse chimeric antibody, and was superior to Tiragolumab (Tira).

### 8.3.3. The binding of the humanized antibody to the TIGIT protein on the cell membrane was studied by flow cytometry

In order to determine whether the humanized TIGIT monoclonal antibody of the present disclosure could bind to TIGIT protein on a cell membrane, *in vitro* testing was performed. Murine antibodies and Tiragolumab (TIRA) were used as positive controls.

The experimental method was the same as in Example 3.2.

### Experimental results and analysis

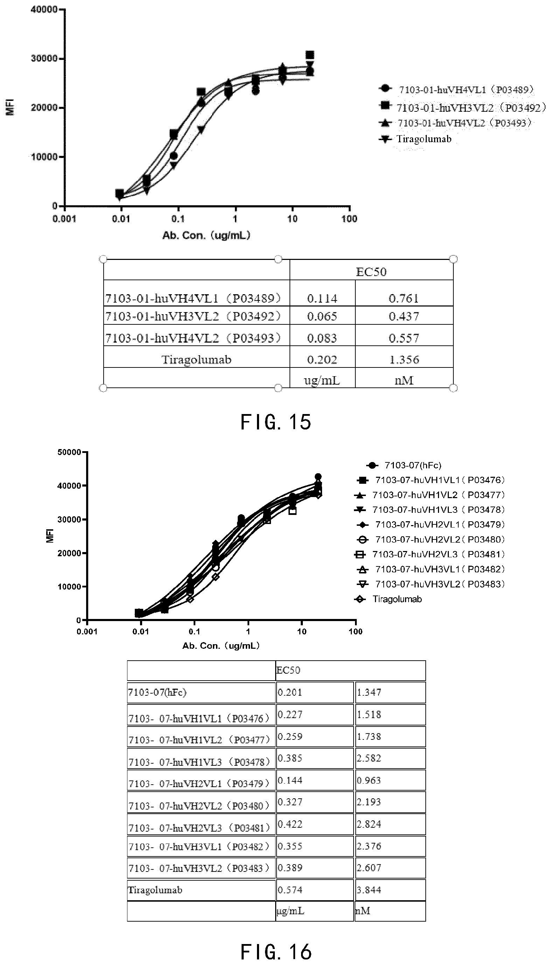

As shown in Fig. 15, three strains of 7103-01 anti-human TIGIT humanized monoclonal antibodies (numbered P03489, P03492, P03493) could bind well to CHO-TIGIT. The EC₅₀ values on the CHO-TIGIT cell membrane were: 0.761 nM, 0.437 nM, 0.557 nM, respectively, which were significantly better than 1.356 nM of Tira.

As shown in Fig. 16, eight strains of 7103-07 anti-human TIGIT humanized monoclonal antibodies (numbered P03476, P03477, P03478, P03479, P03480, P03481, P03482, P03483) could bind well to CHO-TIGIT. The EC₅₀ values on the CHO-TIGIT cell membrane were: 1.518 nM, 1.738 nM, 2.582 nM, 0.963 nM, 2.193 nM, 2.824 nM, 2.376 nM, and 2.607 nM, respectively. Compared to 1.347 nM of human mouse chimeric monoclonal antibodies and 3.844nM of Tira, the humanized antibodies of the present invention had higher affinity.

The humanized antibody of the present disclosure not only had a strong binding capacity with antigen on a solid-phase (ELISA method), but also had a very high binding activity to TIGIT on a cell membrane, and was also superior to the binding of Tira and TIGIT on the surface of the cell membrane.

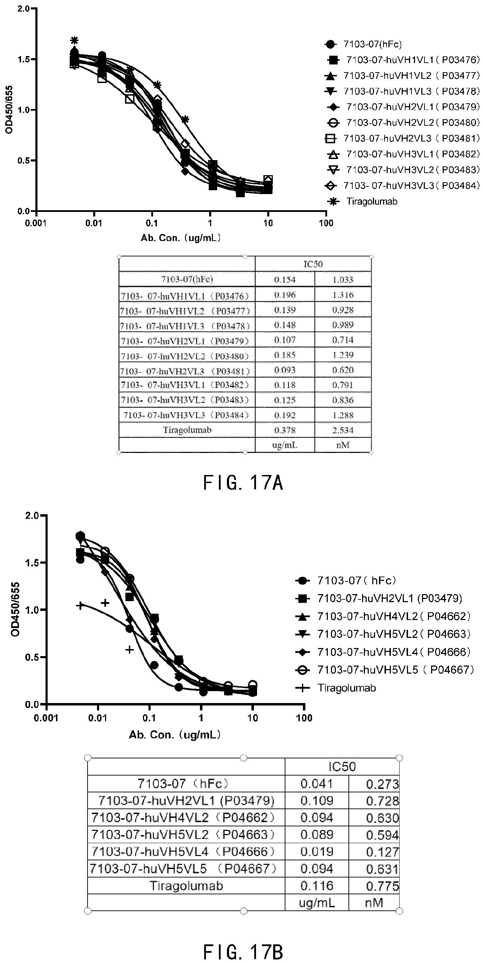

Experimental results (Fig. 17A) showed that 9 strains of 7103-07 anti-human TIGIT humanized monoclonal antibodies (numbered P03476, P03477, P03478, P03479, P03480, P03481, P03482, P03483, and P03484) could specifically block the binding of CD155 to human TIGIT. The IC₅₀ values were 1.316 nM, 0.928 nM, 0.989 nM, 0.714 nM, 1.239 nM, 0.620 nM, 0.791 nM, 0.836 nM, and 1.288 nM, respectively. The blocking capability was equivalent to or better than that of human mouse chimeric monoclonal antibody (IC₅₀ value of 1.033 nM), and was significantly better than Tiragolumab (IC₅₀ value was 2.534 nM).

Experimental results (Fig. 17B) showed that another batch of four strains of 7103-07 anti-human TIGIT humanized monoclonal antibodies (numbered P04662, P04663, P04666 and P04667) could specifically block the binding of CD155 to human TIGIT, and the first batch of P03479 was used as a control. The IC₅₀ values were 0.630nM, 0.594nM, 0.127nM, and 0.631nM, respectively. The blocking ability was equivalent to that of human mouse chimeric monoclonal antibody (IC₅₀ value of 0.273nM), and was better than that of Tiragolumab (IC₅₀ value of 0.775nM).

### Example 9. In Vitro Enhancement of INFγ Generation of PBMC

### 9.1 In Vitro Enhancement of INFγ Generation of PBMC

After T cell activation, lymphocyte secretes cytokine IFN γ. In order to test the function of the humanized TIGIT monoclonal antibody as a positive regulator for T cell activation, the following experiments were performed to demonstrate the enhancement of the antibody of the present disclosure on IFN γ production of PBMC cell via blocking TIGIT signal.

Experimental Methods: frozen human peripheral blood mononuclear cell PBMC (Oricells) was recovered, the cell density was adjusted to 5×10⁶/mL, the antibody was diluted with RPMI 1640 +10% FBS medium to a concentration of 20 µg/mL, PHA concentration was 6 µg/mL, 50 µl of PHA dilution and 50 µl of antibody dilution were added to a 96-well plate respectively, and finally 100 µl of cell suspension was added to a 96-well plate and gently mixed, and cultured in a incubator at 37°C and 5% CO₂ for 4 days. After 4 days, 100 µl supernatant was sucked up for detecting the content of IFNγ.

### Experimental results and analysis

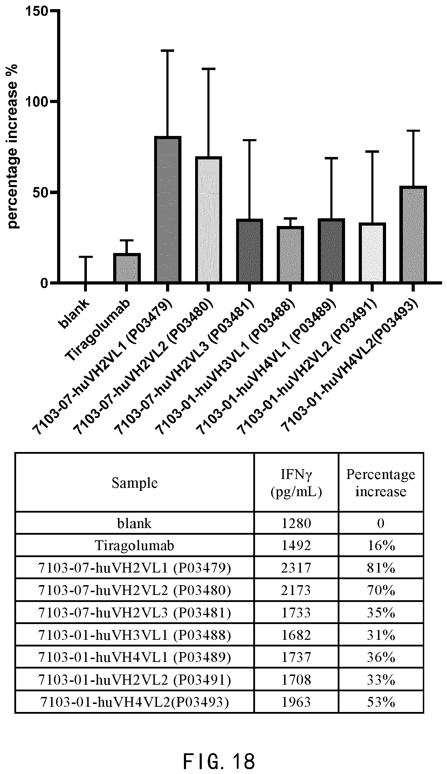

As shown in Fig. 18, 7 strains of humanized TIGIT monoclonal antibodies (numbered P03479, P03480, P03481, P03488, P03489, P03491, and P03493) promoted PHA-activated PBMC to release IFNγ. IFNγ release with the addition of the humanized TIGIT antibody was 31%-81% higher than that without the addition of the humanized TIGIT monoclonal antibody, and the effects were superior to that of Tiragolumab (16%).

### Example 10. Study on the Function of Inhibiting Mouse Tumor Growth in vivo

Since the antibody of the present invention did not bind to mouse TIGIT, we knocked human TIGIT gene in the mouse (C57BL/6) to study the *in vivo* anti-tumor effect of the antibody. Human TIGIT gene knock-in mice were raised in an SPF-level environment. In this example, the mouse tumor model was colon cancer MC 38.

The experimental methods were briefly described as follows: mouse colon cancer MC38 tumor cells were from Shanghai Model Organisms Center, Inc. 24 human TIGIT gene knock-in C57BL/6 mice (7-9 weeks old) were divided into three groups (4 male + 4 female/group), and MC38 cells 1×10⁶/mouse were injected subcutaneously into the armpits of the mice. Each group of mice PBS (control group), recombinant human IgG1 10 mg/kg (control group) and TIGIT humanized antibody P03479 10 mg/kg were given by intraperitoneal injection on the day of inoculation (D0), respectively. The drug was administered twice a week for 4 times. The tumor volume (i.e., long diameter x short diameter²/2) was measured every time the drug was administered, and the weight of the mouse was weighed. At D17, the experiment was ended. The mice were sacrificed by cervical dislocation, and the tumor was weighed.

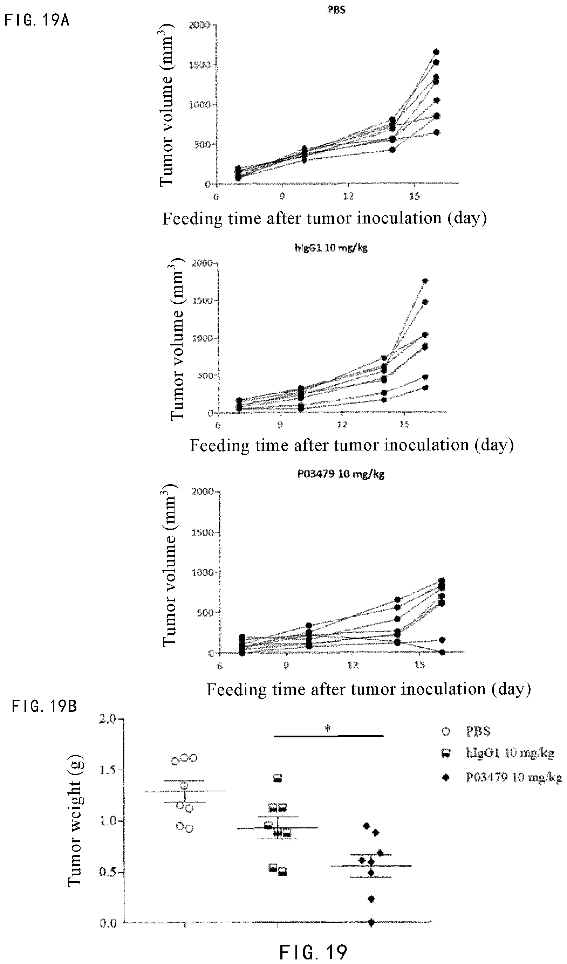

The experimental results were shown in Fig. 19. Fig. 19A showed the relationship between the tumor volume and time for each mouse in each group, and Fig. 19B showed the tumor weight for each mouse in each group at the end of the experiment (D17). Experimental results showed that the TIGIT humanized antibody P03479 could effectively inhibit the growth of tumor in mice (FIGs.19A and 19B), and when the experiment ended (D17), the average weight of the mice tumor in the P03479 group was significantly smaller than that of the hIgG1 group (P < 0.05).

All references mentioned in the present disclosure are incorporated by reference herein, as though individually incorporated by reference. In addition, it should be understood that after reading the above teaching content of the present disclosure, various changes or modifications may be made by those skilled in the art, and these equivalents also fall within the scope as defined by the appended claims of the present disclosure.

## Claims

1. A monoclonal antibody or antigen-binding fragment thereof targeting T cell immunoglobulin and ITIM domain protein (TIGIT), which competes with CD155 to bind to TIGIT, and has a binding affinity EC₅₀ with human TIGIT of 0.01-20nM.

2. The monoclonal antibody or antigen-binding fragment thereof targeting T cell immunoglobulin and ITIM domain protein (TIGIT), wherein the monoclonal antibody has a heavy chain complementary determining region (VH CDR) 1-3 and a light chain complementary determining region (VL CDR) 1-3 selected from the following group, or having a sequence with at least 95% sequence identity thereto:
VH CDR1 selected from the following group consisting of: SEQ ID NO: 28, 36, 44, 52, 60, 68, 76, and 84;
VH CDR2 selected from the following group consisting of: SEQ ID NO: 29, 37, 45, 53, 61, 69, 77, 85, 128, 133, 142, and 143;
VH CDR3 selected from the following group consisting of: SEQ ID NO: 30, 38, 46, 54, 62, 70, 78, and 86;
VL CDR1 selected from the following group consisting of: SEQ ID NO: 32, 40, 48, 56, 64, 72, 80, and 88;
VL CDR2 selected from the following group consisting of: SEQ ID NO: 33, 41, 49, 57, 65, 73, 81, and 89; and
VL CDR3 selected from the following group consisting of: SEQ ID NO: 34, 42, 50, 58, 66, 74, 72, and 90; and/or
the amino acid sequences of VH CDR 1-3 are respectively as follows: SEQ ID NO: 28-30; SEQ ID NO: 36-38; SEQ ID NO: 44-46; SEQ ID NO: 52-54; SEQ ID NO: 60-62; SEQ ID NO: 68-70; SEQ ID NO: 76-78; SEQ ID NO: 84-86; SEQ ID NO: 28, 142, 30; SEQ ID NO: 28, 128, 30; SEQ ID NO: 76, 133, 78; SEQ ID NO: 76, 143, 78; the amino acid sequences of VL CDR 1-3 are respectively as follows: SEQ ID NO: 32-34; SEQ ID NO: 40-42; SEQ ID NO: 48-50; SEQ ID NO: 56-58; SEQ ID NO: 64-66; SEQ ID NO: 72-74; SEQ ID NO: 80-82; SEQ ID NO: 88-90; and/or
the monoclonal antibody has a heavy chain complementary determining region (VH CDR) 1-3 and a light chain complementary determining region (VL CDR) 1-3 selected from the group consisting of:
(a) the amino acid sequences of VH CDR 1-3 are respectively as shown in SEQ ID NO: 28-30, and the amino acid sequences of VL CDR 1-3 are respectively as shown in SEQ ID NO: 32-34;
(b) the amino acid sequences of VH CDR 1-3 are respectively as shown in SEQ ID NO: 36-38, and the amino acid sequences of VL CDR 1-3 are respectively as shown in SEQ ID NO: 40-42;
(c) the amino acid sequences of VH CDR 1-3 are respectively as shown in SEQ ID NO: 44-46, and the amino acid sequences of VL CDR 1-3 are respectively as shown in SEQ ID NO: 48-50;
(d) the amino acid sequences of VH CDR 1-3 are respectively as shown in SEQ ID NO: 52-54, and the amino acid sequences of VL CDR 1-3 are respectively as shown in SEQ ID NO: 56-58;
(e) the amino acid sequences of VH CDR 1-3 are respectively as shown in SEQ ID NO: 60-62, and the amino acid sequences of VL CDR 1-3 are respectively as shown in SEQ ID NO: 64-66;
(f) the amino acid sequences of VH CDR 1-3 are respectively as shown in SEQ ID NO: 68-70, and the amino acid sequences of VL CDR 1-3 are respectively as shown in SEQ ID NO: 72-74;
(g) the amino acid sequences of VH CDR 1-3 are respectively as shown in SEQ ID NO: 76-78, and the amino acid sequences of VL CDR 1-3 are respectively as shown in SEQ ID NO: 80-82;
(h) the amino acid sequences of VH CDR 1-3 are respectively as shown in SEQ ID NO: 84-86, and the amino acid sequences of VL CDR 1-3 are respectively as shown in SEQ ID NO: 88-90;
(i) the amino acid sequences of VH CDR 1-3 are respectively as shown in SEQ ID NO: 28, 142, and 30, and the amino acid sequences of VL CDR 1-3 are respectively as shown in SEQ ID NO: 32-34;
(j) the amino acid sequences of VH CDR 1-3 are respectively as shown in SEQ ID NO: 28, 128, and 30, and the amino acid sequences of VL CDR 1-3 are respectively as shown in SEQ ID NO: 32-34;
(k) the amino acid sequences of VH CDR 1-3 are respectively as shown in SEQ ID NO: 76, 133, and 78, and the amino acid sequences of VL CDR 1-3 are respectively as shown in SEQ ID NO: 80-82;
(l) the amino acid sequences of VH CDR 1-3 are respectively as shown in SEQ ID NO: 76, 143, and 78, and the amino acid sequences of VL CDR 1-3 are respectively as shown in SEQ ID NO: 80-82.

3. A monoclonal antibody or antigen binding fragment thereof of claim 1 or 2, wherein the monoclonal antibody has a heavy chain variable region (VH) and a light chain variable region (VL) sequence selected from the following group consisting of:
a VH amino acid sequence as shown in any one of SEQ ID NO: 27, 35, 43, 51, 59, 67, 75 and 83, or a sequence having at least 70% sequence identity thereto; and a VL amino acid sequence as shown in any one of SEQ ID NO: 31, 39, 47, 55, 63, 71, 79 and 87, or a sequence having at least 70% sequence identity thereto;
for example, the monoclonal antibody has VH and VL sequences selected from the following group consisting of:
(A) a VH amino acid sequence as shown in SEQ ID NO: 27, and a VL amino acid sequence as shown in SEQ ID NO: 31, or a sequence having at least 70% sequence identity thereto;
(B) a VH amino acid sequence as shown in SEQ ID NO: 35, and a VL amino acid sequence as shown in SEQ ID NO: 39, or a sequence having at least 70% sequence identity thereto;
(C) a VH amino acid sequence as shown in SEQ ID NO: 43, and a VL amino acid sequence as shown in SEQ ID NO: 47, or a sequence having at least 70% sequence identity thereto;
(D) a VH amino acid sequence as shown in SEQ ID NO: 51, and a VL amino acid sequence as shown in SEQ ID NO: 55, or a sequence having at least 70% sequence identity thereto;
(E) a VH amino acid sequence as shown in SEQ ID NO: 59, and a VL amino acid sequence as shown in SEQ ID NO: 63, or a sequence having at least 70% sequence identity thereto;
(F) a VH amino acid sequence as shown in SEQ ID NO: 67, and a VL amino acid sequence as shown in SEQ ID NO: 71, or a sequence having at least 70% sequence identity thereto;
(G) a VH amino acid sequence as shown in SEQ ID NO: 75, and a VL amino acid sequence as shown in SEQ ID NO: 79, or a sequence having at least 70% sequence identity thereto; and
(H) a VH amino acid sequence as shown in SEQ ID NO: 83, and a VL amino acid sequence as shown in SEQ ID NO: 87, or a sequence having at least 70% sequence identity thereto.

4. The monoclonal antibody or antigen binding fragment thereof of claim 1 or 2, wherein the monoclonal antibody is a humanized antibody, such as a chimeric antibody, a CDR graft antibody, and a surface remodeling antibody.

5. The monoclonal antibody or antigen binding fragment thereof of claim 4, wherein the monoclonal antibody is a chimeric antibody, wherein,
its constant region (C region) is a human constant region, for example, its heavy chain constant region is human IgG (such as IgG1, IgG2, IgG3 or IgG4) and/or its light chain constant region is human x or λ;
its variable region (V region) is a mouse variable region, for example, the chimeric antibody further has VH and VL sequences of claim 2 or VH CDR 1-3 and VL CDR 1-3 sequences of claim 3; or
any pair of corresponding paired primers selected from SEQ ID NO: 91-123 are used in the construction of the chimeric antibody expression vector.

6. The monoclonal antibody or antigen binding fragment thereof of claim 1 or 2, wherein the monoclonal antibody is a CDR graft antibody, which comprises the VH CDR 1-3 and VL CDR 1-3 of claim 3.

7. A monoclonal antibody or antigen binding fragment thereof of claim 1 or 2, wherein the monoclonal antibody is a surface remodeling antibody, wherein the monoclonal antibody is obtained by humanization design and substitution of one or more amino acid residues in VH and VL by the monoclonal antibody of claim 2, the amino acid substitution is located or not located in CDR (for example, VH CDR2 of SEQ ID NO: 29 is replaced by humanized VH CDR2 of SEQ ID NO: 128 or SEQ ID NO: 142; VL CDR2 of SEQ ID NO: 77 is replaced by humanized VL CDR2 of SEQ ID NO: 133 or SEQ ID NO: 143);
for example, the monoclonal antibody has VH and VL sequences selected from the following group consisting of:
a VH amino acid sequence as shown in any one of SEQ ID NO: 124, 125, 126, 127, 131, 132, 134, 135 and 136, or a sequence having at least 70% sequence identity thereto; and/or a VL amino acid sequence as shown in any one of SEQ ID NO: 129, 130, 137, 138, 139, 140 and 141, or a sequence having at least 70% sequence identity thereto;
for example, the monoclonal antibody has VH and VL sequences selected from the following group consisting of:
(A') a VH amino acid sequence as shown in SEQ ID NO: 124 or a sequence having at least 70% sequence identity thereto; and a VL amino acid sequence as shown in SEQ ID NO: 129 or a sequence having at least 70% sequence identity thereto;
(B') a VH amino acid sequence as shown in SEQ ID NO: 125 or a sequence having at least 70% sequence identity thereto; and a VL amino acid sequence as shown in SEQ ID NO: 129 or a sequence having at least 70% sequence identity thereto;
(C') a VH amino acid sequence as shown in SEQ ID NO: 126 or a sequence having at least 70% sequence identity thereto; and a VL amino acid sequence as shown in SEQ ID NO: 129 or a sequence having at least 70% sequence identity thereto;
(D') a VH amino acid sequence as shown in SEQ ID NO: 127 or a sequence having at least 70% sequence identity thereto; and a VL amino acid sequence as shown in SEQ ID NO: 129 or a sequence having at least 70% sequence identity thereto;
(E') a VH amino acid sequence as shown in SEQ ID NO: 124 or a sequence having at least 70% sequence identity thereto; and a VL amino acid sequence as shown in SEQ ID NO: 130 or a sequence having at least 70% sequence identity thereto;
(F') a VH amino acid sequence as shown in SEQ ID NO: 125 or a sequence having at least 70% sequence identity thereto; and a VL amino acid sequence as shown in SEQ ID NO: 130 or a sequence having at least 70% sequence identity thereto;
(G') a VH amino acid sequence as shown in SEQ ID NO: 126 or a sequence having at least 70% sequence identity thereto; and a VL amino acid sequence as shown in SEQ ID NO: 130 or a sequence having at least 70% sequence identity thereto;
(H') a VH amino acid sequence as shown in SEQ ID NO: 127 or a sequence having at least 70% sequence identity thereto; and a VL amino acid sequence as shown in SEQ ID NO: 130 or a sequence having at least 70% sequence identity thereto;
(I') a VH amino acid sequence as shown in SEQ ID NO: 131 or a sequence having at least 70% sequence identity thereto; and a VL amino acid sequence as shown in SEQ ID NO: 137 or a sequence having at least 70% sequence identity thereto;
(J') a VH amino acid sequence as shown in SEQ ID NO: 131 or a sequence having at least 70% sequence identity thereto; and a VL amino acid sequence as shown in SEQ ID NO: 138 or a sequence having at least 70% sequence identity thereto;
(K') a VH amino acid sequence as shown in SEQ ID NO: 131 or a sequence having at least 70% sequence identity thereto; and a VL amino acid sequence as shown in SEQ ID NO: 139 or a sequence having at least 70% sequence identity thereto;
(L') a VH amino acid sequence as shown in SEQ ID NO: 132 or a sequence having at least 70% sequence identity thereto; and a VL amino acid sequence as shown in SEQ ID NO: 137 or a sequence having at least 70% sequence identity thereto;
(M') a VH amino acid sequence as shown in SEQ ID NO: 132 or a sequence having at least 70% sequence identity thereto; and a VL amino acid sequence as shown in SEQ ID NO: 138 or a sequence having at least 70% sequence identity thereto;
(N') a VH amino acid sequence as shown in SEQ ID NO: 132 or a sequence having at least 70% sequence identity thereto; and a VL amino acid sequence as shown in SEQ ID NO: 139 or a sequence having at least 70% sequence identity thereto;
(O') a VH amino acid sequence as shown in SEQ ID NO: 134 or a sequence having at least 70% sequence identity thereto; and a VL amino acid sequence as shown in SEQ ID NO: 137 or a sequence having at least 70% sequence identity thereto;
(P') a VH amino acid sequence as shown in SEQ ID NO: 134 or a sequence having at least 70% sequence identity thereto; and a VL amino acid sequence as shown in SEQ ID NO: 138 or a sequence having at least 70% sequence identity thereto; or
(Q') a VH amino acid sequence as shown in SEQ ID NO: 134 or a sequence having at least 70% sequence identity thereto; and a VL amino acid sequence as shown in SEQ ID NO: 139 or a sequence having at least 70% sequence identity thereto;
(R') a VH amino acid sequence as shown in SEQ ID NO: 135 or a sequence having at least 70% sequence identity thereto; and a VL amino acid sequence as shown in SEQ ID NO: 138 or a sequence having at least 70% sequence identity thereto;
(S') a VH amino acid sequence as shown in SEQ ID NO: 136 or a sequence having at least 70% sequence identity thereto; and a VL amino acid sequence as shown in SEQ ID NO: 138 or a sequence having at least 70% sequence identity thereto;
(T') a VH amino acid sequence as shown in SEQ ID NO: 135 or a sequence having at least 70% sequence identity thereto; and a VL amino acid sequence as shown in SEQ ID NO: 141 or a sequence having at least 70% sequence identity thereto;
(U') a VH amino acid sequence as shown in SEQ ID NO: 136 or a sequence having at least 70% sequence identity thereto; and a VL amino acid sequence as shown in SEQ ID NO: 140 or a sequence having at least 70% sequence identity thereto;
(V') a VH amino acid sequence as shown in SEQ ID NO: 136 or a sequence having at least 70% sequence identity thereto; and a VL amino acid sequence as shown in SEQ ID NO: 141 or a sequence having at least 70% sequence identity thereto.

8. A monoclonal antibody or antigen binding fragment thereof of claim 1 or 2, which has one or more features selected from the following group consisting of:
(i) specifically binding to human TIGIT *in vivo* or *in vitro,* for example, wherein its binding affinity EC50 with human TIGIT determined by ELISA is 0.001 nM to 100 nM, 0.01 nM to 50 nM, or 0.1 nM to 10 nM;
(ii) competitively inhibiting binding of CD155 to TIGIT, for example, wherein its specific inhibition IC₅₀ of CD155 binding to human TIGIT measured by ELISA is 0.005 µg/ml-1 µg/ml, or 0.01 µg/ml-0.9 µg/ml;
(iii) binding to human or primate mammalian TIGIT without binding to mouse TIGIT;
(iv) positively regulating activity of immune cells (such as T cells and NK cells), such as increasing the production of immune response cytokines (such as IFNγ) of human peripheral blood mononuclear cells (PBMC) (such as lymphocytes, T cells), reducing or reversing NK cell depletion;
(v) reducing or eliminating cells expressing TIGIT, such as Treg cells, reducing immunosuppressive effect;
(vi) enhancing an immune response; and
(vii) having a prophylactic and/or therapeutic effect against tumors, infections, or infectious diseases.

9. A nucleic acid molecule encoding the monoclonal antibody or the antigen-binding fragment thereof of any one of claims 1-8, or a vector or a host cell containing the nucleic acid molecule.

10. An immunoconjugate containing:
(a) the monoclonal antibody or antigen-binding fragment thereof of any one of claims 1-8;
(b) a coupling moiety selected from the group consisting of a drug, a toxin, a cytokine, a radionuclide or an enzyme; and
(c) optionally, a linker.

11. A chimeric antigen receptor, comprising an extracellular domain and an intracellular domain, wherein the extracellular domain comprises the monoclonal antibody or antigen-binding fragment thereof of any one of claims 1-8.

12. The chimeric antigen receptor of claim 11, wherein:
the monoclonal antibody or antigen-binding fragment thereof comprises or consists of scFv or VH sdAb;
the intracellular domain comprises one or more components selected from the following groups: ITAM domain, CD3ζ, CD28, 4-1BB, OX40, CD27, ICOS or a combination thereof, such as (CD28 +CD3ζ), (CD28 +CD27 + CD3ζ), (CD28 + OX40 + CD3ζ), (CD28 + 4-1BB + CD3ζ), (CD28 + CD27 + OX40 + CD3ζ), (CD28 + 4-1BB + CD27 + CD3ζ), (CD28 + 4-1BB + OX40 + CD3ζ), (4-1BB + CD3ζ), (4-1BB + OX40 + CD3ζ), (4-1BB + CD27 +CD3ζ), (CD27 + CD3ζ), (CD27 + OX 40 + CD3ζ), (CD28Δ +CD3ζ), (CD28Δ +CD27 + CD3ζ), (CD28Δ + OX40 + CD3ζ), (CD28Δ + 4-1BB + CD3ζ), (CD28Δ + 4-1BB + OX40 + CD3ζ), (CD28Δ + CD27 + OX40 + CD3ζ), (CD28Δ + 4-1BB + CD27 + CD3ζ)), (4-1BB + ICOS + CD3ζ), (CD28 + ICOS + CD3ζ), (ICOS + CD3ζ); and/or
the extracellular domain of the chimeric antigen receptor further comprises a hinge region, for example, the hinge region is derived from an IgG hinge or a CD8α/CD28 extracellular region, such as selected from the group consisting of: IgG4 Fc Δ EQ, IgG4 Fc Δ Q, (t-12AA + t-20AA), mKate, phiLov, dsRed, Venus, eGFP, CH3 HA, (CD8 α + t-20AA), double t-20 AA, (t-20 AA + CD8α), (CD8α + leucine zipper Basep1), (CD8α+ leucine zipper Acid1), 2D3, CD8α, or IgG4Fc; and/or
the chimeric antigen receptor further comprises a transmembrane domain connecting the extracellular domain and the intracellular domain, such as α, β or ζ chain derived from T-cell receptors, such as a transmembrane region of CD28, CD3ε, CD45, CD4, CD5, CDS, CD9, CD16, CD22, CD33, CD37, CD64, CD80, CD86, CD 134, CD137, or CD 154.

13. A nucleic acid molecule and a construct or vector comprising the nucleotide molecule, wherein the nucleic acid molecule comprises a coding sequence of the chimeric antigen receptor of claim 11 or 12.

14. A transformed immune cell, which expresses the chimeric antigen receptor of claim 11 or 12, or is transformed using a nucleic acid molecule, construct, or vector of claim 13, for example, the immune cell is selected from T cells, such as αβ T cells, γδ T cells or NK T cells or T cells derived from pluripotent cells.

15. A composition comprising: (A) the monoclonal antibody or antigen binding fragment thereof of claims 1-8, the nucleic acid molecule, vector or host cell of claim 9, or the immunoconjugate of claim 10, the chimeric antigen receptor of claims 11 or 12, the nucleic acid molecule, construct or vector of claim 13, and/or a transformed immune cell of claim 14, and (B) a carrier;
for example, the composition is: a pharmaceutical composition, which further comprises a pharmaceutically acceptable carrier; and a detection kit, which further comprises a reagent required for detecting the TIGIT level or its activity or its related pathways.

16. Use of the monoclonal antibody or antigen binding fragment thereof of any one of claims 1-8, the nucleic acid molecule, vector or host cell of claim 9, the immunoconjugate of claim 10, the chimeric antigen receptor of claims 11 or 12, the nucleic acid molecule, construct or vector of claim 13, the transformed immune cells of claim 14 and/or the composition of claim 15 in preparation of a product for positively regulating immune cell activity and/or improving immune response, such as in preparation of a medicine for preventing and/or treating tumor, infection or infectious disease.

17. Use of the monoclonal antibody or antigen binding fragment thereof of any one of claims 1-8, the nucleic acid molecule, vector or host cell of claim 9, the immunoconjugate of claim 10, the chimeric antigen receptor of claims 11 or 12, the nucleic acid molecule, construct or vector of claim 13, the transformed immune cells of claim 14 and/or the composition of claim 15 in preparation of a product for reducing or eliminating the immunosuppressive effects of cells expressing TIGIT (such as Treg cells).

18. Use of the monoclonal antibody or antigen binding fragment thereof of any one of claims 1-8, the nucleic acid molecule, vector or host cell of claim 9, the immunoconjugate of claim 10, the chimeric antigen receptor of claims 11 or 12, the nucleic acid molecule, construct or vector of claim 13, the transformed immune cells of claim 14 and/or the composition of claim 15 in preparation of a kit for detecting the presence, level, or activity of TIGIT.
